Europäisches Patentamt

European Patent Office

Office européen des brevets

(11) Veröffentlichungsnummer: **0 299 402 B1**

# EUROPÄISCHE PATENTSCHRIFT

(45) Veröffentlichungstag der Patentschrift: **09.06.93**

(51) Int. Cl.5: **C07K 7/20, A61K 37/43**

(21) Anmeldenummer: **88111031.6**

(22) Anmeldetag: **11.07.88**

(54) **LHRH Antagonisten, deren Herstellung und entsprechende pharmazeutische Zubereitungen.**

(30) Priorität: **17.07.87 US 74126**

(43) Veröffentlichungstag der Anmeldung:
**18.01.89 Patentblatt 89/03**

(45) Bekanntmachung des Hinweises auf die
Patenterteilung:
**09.06.93 Patentblatt 93/23**

(84) Benannte Vertragsstaaten:
**AT BE CH DE ES FR GB GR IT LI LU NL SE**

(56) Entgegenhaltungen:
**EP-A- 0 081 877**

**Z. NATURFORSCH., Band 42b, 1987, Seiten 101-106; K. FOLKERS et al.: "Activities of antagonists of the luteinizing hormone releasing hormone with emphasis on positions 1,5 and 6 and on positions 1,2 and 3"**

**CHEMICAL ABSTRACTS, Band 82, 1975, Seite 488, Zusammenfassung Nr. 73465h, Columbus, Ohio, US; Y. YABE et al.: "Analogs of luteinizing hormone-releasing hormone with modification in position 8", & CHEM. PHARM. BULL. 1974, 22(11), 2557-64**

**PROC. NATL. ACAD. SCI. USA, Band 85, März**

**1988, Seiten 1637-1641; S. BAJUSZ et al.: "Highly potent antagonists of luteinizing hormone-releasing hormone free of edematogenic effects"**

**CHEMICAL ABSTRACTS, Band 110, 1989, Seite 94, Zusammenfassung Nr. 186130k, Columbus, Ohio, US; S. BAJUSZ et al.: "New antagonists of LH-RH. II. Inhibition and potentiation of LH-RH by closely related analogs", & INT. J. PEPT. PROTEIN RES. 1988, 32(6), 425-35**

(73) Patentinhaber: **ASTA Medica Aktiengesellschaft**
**Weismüllerstrasse 45**
**W-6000 Frankturt am Main 1(DE)**

(72) Erfinder: **Schally, Andrew V., Prof.**
**5025 Kawanee Avenue**
**Metairie, LA 70002(US)**
Erfinder: **Bajusz, Sandor, Dr.**
**10501 Curran Boulevard No. 5W**
**New Orleans, LA 70127(US)**

EP 0 299 402 B1

## Beschreibung

Die vorliegende Erfindung bezieht sich auf neue Peptide, die die Freisetzung von Gonadotropinen durch die Hirnanhangsdrüse bei Säugetieren hemmen, ohne jedoch oedematöse Reaktionen hervorzurufen. Insbesondere bezieht sich die vorliegende Erfindung auf Analoge des das luteinisierende Hormon freisetzende Hormons (LHRH), das die folgende Struktur aufweist:

$$\overset{1}{p-Glu}-\overset{2}{His}-\overset{3}{Trp}-\overset{4}{Ser}-\overset{5}{Tyr}-\overset{6}{Gly}-\overset{7}{Leu}-\overset{8}{Arg}-\overset{9}{Pro}-Gly-NH_2,$$

Salze davon und pharmazeutische Verbindungen und Anwendungsverfahren, die diese Analoge betreffen.

Während mehr als 15 Jahren haben Forscher nach selektiv potenten Antagonisten des LHRH-Dekapeptids [M. Karten und J.E. Rivier, Endocrine Reviews, 7, 44-66 (1986)] gesucht. Das hohe Interesse an solchen Antagonisten ist in ihrer Nützlichkeit im Bereich der Endokrinologie, Gynäkologie, Schwangerschaftsverhütung und Krebs begründet. Eine grosse Anzahl von Verbindungen sind als potentielle LHRH-Antagonisten hergestellt worden. Die interessantesten Antagonisten, die bis heute gefunden worden sind, sind jene Verbindungen, deren Struktur eine Modifizierung der LHRH-Struktur darstellen.

Die erste Serie von potenten Antagonisten wurde durch die Einführung von aromatischen Aminosäureresten in den Stellungen 1, 2, 3 und 6 oder 2, 3 und 6 erhalten. Die Verbindungen werden beschrieben als LHRH, das durch das Ersetzen der ursprünglichen Aminosäureresten durch andere an den durch die hochgestellten Nummern bezeichnet wird. Die bekannten Antagonisten umfassen:

[Ac-D-Phe(4-Cl)[1,2], D-Trp[3,6]] LHRH (D.H. Coy, et. al., In: Gross, E. and Meienhofer, J. (Eds) Peptides, Proceedings of the 6th American Peptid Symposium, S. 775-779, Pierce Chem.Co., Rockville, Il.,1979);

[Ac-Pro[1], D-Phe(4-Cl)[2], D-Nal(2)[3,6]] LHRH (US Patent Nr. 4,419,347) und

[Ac- [3]Pro[1],D-Phe(4-Cl)[2], D-Trp[3,6]]LHRH (J.L. Pineda, et al., J. Clin. Endocrinol. Metab. 56, 420, 1983).

Um die Wasserlöslichkeit der Antagonisten zu erhöhen, wurden später basische Aminosäuren, wie zum Beispiel D-Arg, in der 6-Stellung eingeführt. Zum Beispiel [Ac-D-Phe(4-Cl)[1,2], D-Trp[3], D-Arg[6], D-Ala[10]] LHRH (ORG-30276) (D.H. Coy, et al., Endocrinology, 100, 1445, 1982); und

[Ac-D-Nal(2)[1], D-Phe(4-F)[2], D-Trp[3], D-Arg[6]]LHRH (ORF 18260) (J.E. Rivier, et al., In: Vickery B.H., Nestor, Jr. J.J., Hafez, E.S.E. (Eds), LHRH and Its Analogs, S. 11-22 MTP Press, Lancaster, UK, 1984).

Solche wasserlöslichen LH-RH-Antagonisten mit einem basischen Aminosäurerest in der 6-Stellung sind auch Gegenstand der Europa-Anmeldung 81 877 wobei die basischen Aminosäuren D-Lys, D-Arg, D-Orn, D-homo-Arg oder D-His sind. Ebenso werden LH-RH-Antagonisten mit einem basischen Aminosäurerest in 6-Stellung und vorzugsweise einem basischen Aminosäurerest, der sich von D-3-Pal ableitet, in der 3-Stellung, in der Zeitschrift für Naturforschung, Band 426, 1987, Seiten 101 - 106 beschrieben.

Diese Analoge weisen nicht nur die erwartete verbesserte Wasserlöslichkeit auf sondern zeigten auch eine verbesserte antagonistische Wirksamkeit. Dennoch verursachen diese äußerst potenten, hydrophilischen Analoge mit D-Arg und anderen basischen Seitenketten an der 6 Stellung, vorübergehende Oedeme auf dem Gesicht und den Extremitäten zu bilden, wenn sie Ratten in Dosen von 1,25 oder 1,5 mg/kg subkutan verabreicht wurden (F. Schmidt, et al., Contraception, 29, 283, 1984; J.E. Morgan, et al., Int. Archs. Allergy Appl. Immun. 80, 70, (1986). Da das Auftreten von oedematogenen Auswirkungen nach der Verabreichung dieser Antagonisten bei Ratten Zweifel über ihre Sicherheit bei der Verwendung bei Menschen aufkommen liessen und so die Einführung dieser Arzneimittel in die klinische Verwendung verzögerten, ist es wünschenswert, antagonistische Peptide vorzusehen, die frei von diesen Nebenwirkungen sind.

Die vorliegende Erfindung bezieht sich auf LHRH-Antagonisten, die eine verbesserte Wasserlöslichkeit und hohe antagonistische Potenz der basischen Peptide aufweisen und frei von oedematogenen Wirkungen sind. Diese Verbindungen sind hochpotent bei der Hemmung der Freisetzung von Gonadotropinen aus der Hirnanhangsdrüse bei Säugetieren, einschliesslich bei Menschen.

Die Verbindungen der vorliegenden Erfindung werden durch Formel I dargestellt:

X-R[1]-R[2]-R[3]-Ser-Tyr-R[6]-Leu-Arg-Pro-R[10]-NH₂    I

worin

X eine Acylgruppe, von geraden oder verzweigten Ketten aliphatischer oder alicyklischer Carbonsäuren mit 1 bis 7 Kohlenstoffatomen, oder eine Carbamoylgruppe ist,
R[1] D- oder L-Pro, D- oder L-Δ[3]-Pro, D-Phe, D-Phe(4-Hl), D-Ser, D-Thr, D-Ala, D-Nal(1) oder D-Nal(2) ist,

$R^2$ D-Phe oder D-Phe(4-Hl) ist,

$R^3$ D-Trp, D-Phe, D-Pal(3), D-Nal(1) oder D-Nal(2) ist,

$R^6$ D-Cit, D-Hci, D-Cit(Q) oder D-Hci(Q)

$R^{10}$ Gly oder D-Ala ist,

wobei Hl Fluor, Chlor oder Brom und Q $C_1$-$C_3$-Alkyl ist, und pharmazeutisch annehmbaren Säureadditionssalze davon.

Die Verbindungen der Formel I werden nach den bekannten Methoden synthetisiert, wie zum Beispiel durch reine Festphasentechnik, teilweise Festphasentechnik oder durch die klassischen Lösungskupplungen. Die verbindungen der Formel I werden vorzugsweise durch eine bekannte Festphasentechnik hergestellt. Eine derartige Methode liefert Zwischenpeptide beziehungsweise Zwischenpeptidharze der Formel II:

$$X_1 \text{-} R^1 \text{-} R^2 \text{-} R^3 \text{-} Ser(X^4) \text{-} Tyr(X^5) \text{-} R^{*6} \, Leu \text{-} Arg(X^5) \text{-} Pro \text{-} R^{10} \text{-} NH \text{-} X^{10} \qquad II$$

worin $X_1$ eine Acylgruppe ist, basierend auf geraden und verzweigten Ketten aliphatischer oder alicyclischer Carbonsäuren mit 1 bis 7 Kohlenstoffatomen (insbesondere Acetyl), t-Boc, Wasserstoff oder Carbamoyl, $X^4$ eine Schutzgruppe für die Ser-Hydroxygruppe ist (zum Beispiel Benzyl, 2,6-Dichlor-benzyl), $X^5$ eine Schutzgruppe für die Tyr-phenolische Hydroxylgruppe ist (zum Beispiel Benzyl, 2,6-Dichlor-benzyl, 2-Chlor-(2-brom)-benzyloxycarbonyl; Benzyloxycarbonyl).

$X^5$ eine Schutzgruppe für die Arg-Guanidinogruppe ist ($NO_2$, Tos).

$X^{10}$ ein Benzhydryl- oder Methylbenzhydrylgruppen erhaltendes Trägerharz ist,

$R^1$ D- oder L-Pro, D- oder L-$\Delta^3$-Pro, D-Phe, D-Phe(4-Hl), D-Ser, D-Thr, D-Ala, D-Nal(1) oder D-Nal(2) ist,

$R^2$ D-Phe oder D-Phe(4-Hl) ist,

$R^3$ D-Trp, D-Phe, D-Pal(3), D-Nal(1) oder D-Nal(2) ist.

$R^{*6}$ D-Cit, D-Hci, D-Cit(Q) oder D-Hci(Q) und $R^{10}$ Gly oder D-Ala ist.

wobei Hl Fluor, Chlor oder Brom ist,

Eine Verfahrensweise besteht beispielsweise in der Umsetzung eines Peptides der Formel II, worin $R^{*6}$ D-Lys oder D-Orn und $X^5$ Wasserstoff sind, mit einem Cyanat oder einer Verbindung die Cyanat liefert (Cyanat-Quelle) zu einem Peptid der Formel III:

$$X_1 \text{-} R^1 \text{-} R^2 \text{-} R^3 \text{-} Ser(X^4) \text{-} Tyr(X^5) \text{-} R^{**6} \text{-} Leu \text{-} Arg(X^5) \text{-} Pro \text{-} R^{10} \text{-} NH \text{-} X^{10} \qquad III$$

worin $X_1$, $R^1$, $R^2$, $R^3$, $X^4$, $X^5$, $X^5$, $R^{10}$ und $X^{10}$ die angegebenen Bedeutungen haben und $R^{**6}$ Cit, Hci, Cit(Q) oder Hci(Q) ist. Vorzugsweise wird diese Reaktion ausgeführt, wenn $X_1$ Acyl ist und alle anderen Reste X Wasserstoff sind.

Geeignete Cyanate beziehungsweise cyanatliefernde Verbindungen sind: Alkalicyanate (zum Beispiel Kaliumcyanat), N-niederalkyl-isocyanate (N-ethylisocyanat). Die Peptide der Formel II werden vorzugsweise nach der bekannten Festphasentechnik synthetisiert. Die Umsetzung mit Cyanaten beziehungsweise cyanatliefernde Verbindungen wird in Lösungsmitteln, vorzugsweise aprotischen Mitteln wie zum Beispiel wässrigen DMF bei Temperaturen zwischen 0 und 50° C, vorzugsweise 0 und 25° C durchgeführt.

Der Fortgang dieser Reaktion, das heißt die Überführung von Orn/Lys-Peptiden in die entsprechenden Cit/Hci-Peptide kann leicht verfolgt werden durch HPLC in MeCN-wässrigen TFA-Systemen, infolge eines charakteristischen 2,6 ± 0,3 Minuten-Ansteigens der Retensionszeit von Cit/Hci- und zum Beispiel Cit(Ethyl)-/Hci(Ethyl)-Peptiden im Vergleich zu den entsprechenden Orn/Lys-Peptiden.

Peptide der Formel I worin X eine Acylgruppe ist, werden direkt durch Spaltung und Entschützen der Zwischenpeptidharze der Formel II erhalten, worin $X_1$ eine Acylgruppe und $R^{*6}$ D-Cit, D-Hci, D-Cit(Q) oder D-Hci(Q), Peptide der Formel I, worin X eine Carbamoyl-Gruppe ist, werden aus Peptidharzen der Formel II erhalten, worin $X_1$ Wasserstoff oder Boc ist, und zwar durch Spaltung und Entschützen mit anschliessender Carbamoylierung.

Eine Gonadotropin antagonisierende, pharmazeutische Zusammensetzung wird durch Mischen der Verbindung der Formel I mit einem pharmazeutisch annehmbaren Träger einschliesslich Mikrokügelchen für die verzögerte Abgabe vorgesehen.

Es ist ebenfalls ein Verfahren vorgesehen, durch das Komplikationen erleichtert werden, die sich aus der physiologischen Verfügbarkeit von Mengen von Gonadotropinen aus der Hirnanhangsdrüse beim Säugetier ergeben, die über der gewünschten Menge liegen, was die Verabreichung an ein Säugetier einer Gonadotropin antagonisierenden Dosis der Verbindung der Formel I umfasst.

Die zur Definierung der Peptide verwendete Nomenklatur stimmt mit jener durch die IUPAC-IUB-Kommission über Biochemische Nomenklatur erläuterten Nomenklatur überein (European J. Biochem., 1984, 138, 9-37), worin in Uebereinstimmung mit der herkömmlichen Darstellung die Aminogruppen beim

3

N-Terminus nach links erscheinen und die Carboxylgruppe beim C-Terminus nach rechts. Der Ausdruck natürliche Aminosäuren wird allgemein für natürlich auftretende Aminosäuren verwendet, die in Proteinen gefunden werden, die Gly, Ala, Val, Leu, Ile, Ser, Thr, Lys, Arg, Asp, Asn, Glu, Gln, Cys, Met, Phe, Tyr, Pro Trp und His umfassen. Die Abkürzungen für die einzelnen Aminosäurereste beruhen auf dem Trivialname der Aminosäure und sind Ala, Alanin; Arg, Arginin; Cit, Citrullin; Gly, Glycin; Hci, Homocitrullin; Leu, Leucin; Lys, Lysin; Pal(3), 3-(3-Pyridyl)alanin; Nal(2), 3-(2-Naphtyl)alanin; Nal(1), 3-(1-naphthyl)alanin; Orn, Ornithin; Phe, Phenylalanin; Phe(4-Cl), 4-Chlorphenylalanin; Phe(4-F), 4-Fluorophenylalanin; Pro, Prolin; Ser, Serin; Trp, Tryptophan und Tyr, Tyrosin. Alle hier beschriebenen Aminosäuren stammen aus der L-Serie, wenn nicht anders erwähnt, zum Beispiel D-Trp bedeutet D-Tryptophan und D-Nal(2) bedeutet 3-(2-Naphtyl)-D-Alanin. D-Cit(Q) oder D-Hci(Q) bedeuted, daß ein H-Atom der Aminogruppe des Ureidoteils des Citrullins (Homocitrullins) durch die Alkylgruppe Q substituiert ist. Andere verwendete Abkürzungen sind:

| | |
|---|---|
| AcOH | Essigsäure |
| AcOEt | Aethylacetat |
| $Ac_2O$ | Essigsäureanhydrid |
| Boc- | tert. Butyloxycarbonyl |
| DIC | Diisopropylcarbodiimid |
| DIEA | N,N-Diisopropyläthylamin |
| DMF | Dimethylformamid |
| HOBt | 1-Hydroxybenzentriazolhydrat |
| HPLC | Hochdruckflüssigchromatographie |
| MeOH | Methylalkohol |
| TEA | Triäthylamin |
| DCC | Dicyclohexylcarbodiimid |
| MeCN | Acetonitril |
| IpOH | Isopropanol |
| Z(2-Cl) | 2-Chlorbenzyloxycarbonyl |
| DCB | 2,6-Dichlorbenzyl |
| Tos | p-Toluolsulfonyl |
| TFA | Trifluoressigsäure |
| Z | Benzyloxycarbonyl |

Besonders bevorzugt sind die LHRH-Analoge der Formel I, worin:

X Acetyl oder Carbamoyl ist,

$R^1$ D-Phe(4-Cl), D-Nal(2), Pro oder D-Phe ist,

$R^2$ D-Phe(4-Cl) oder D-Phe(4-F) ist,

$R^3$ D-Trp oder D-Pal(3) ist,

$R^6$ D-Cit, D-Hci, D-Cit(Ethyl), D-Hci(Ethyl) ist und

$R^{10}$ D-Ala ist.

Die Herstellung der erfindungsgemäßen Peptide erfolgt dadurch, daß man sie durch in der Peptidchemie übliche Fragmentkondensation von entsprechenden Peptidbruchstücken oder durch in der Peptidchemie üblichen stufenweisen Aufbau herstellt oder daß man in einem Peptid der Formel I, worin $R^6$ D-Orn oder D-Lys ist, die freie Aminogruppe von D-Orn oder D-Lys durch Umsetzung mit einem Alkalicyanat oder einer Cyanatliefernden Verbindung oder einem $C_1$-$C_3$-Alkylisocyanat zur Ureidogruppe umsetzt und gegebenenfalls die erhaltenen Peptide acyliert und/oder in die Amide überführt und gegebenenfalls in physiologisch unbedenkliche Säuresalze überführt.

Der stufenweise Aufbau erfolgt zum Beispiel, indem man zunächst die Carboxy-terminale Aminosäure Glycin oder D-Alanin beziehungsweise deren Amide, deren α-ständige Aminogruppe geschützt ist, an einen hierfür üblichen synthetischen Träger kovalent bindet, die α-Amino-Schutzgruppe abspaltet, an die so erhaltene freie Aminogruppe die nächstfolgende geschützte Aminosäure über ihre Carboxy-Gruppe bindet, dann wiederum die α-Amino-Schutzgruppe dieser zweiten Aminosäure abspaltet, hieran die nächste Aminosäure bindet und in dieser Weise Schritt für Schritt die übrigen Aminosäuren des zu synthetisierenden Peptids in der richtigen Reihenfolge verknüpft und nach Verknüpfung aller Aminosäuren das fertige Peptid gemäß Anspruch 1 vom Träger abspaltet und gegebenenfalls weitere vorhandene Seitenfunktions-Schutzgruppen abspaltet.

Bei der Fragmentkupplung verwendet man vorzugsweise die ohne Racemisierung verlaufende Azidkupplung oder die Dicyclohexylcarbodiimid/1-Hydroxybenzotriazolbeziehungsweise DCC/3-Hydroxy-4-oxo-3,4-dihydro-1,2,3-benzotriazin-Methode. Man kann auch aktivierte Ester von Fragmenten einsetzen (DCC = Dicyclohexylcarbodiimid).

4

Für die stufenweise Kondensation von Aminosäuren eignen sich besonders gut aktivierte Ester von Benzyloxycarbonyl-aminosäuren, wie zum Beispiel N-Hydroxysuccinimidester oder 2,4,5-Trichlorphenylester und 4-Nitrophenylester . Die Aminolyse der beiden letzteren Aktivester läßt sich sehr gut durch N-Hydroxyverbindungen, die in etwa die Acidität der Essigsäure besitzen, wie zum Beispiel 1-Hydroxybenzotriazol katalysieren.

Als intermediäre Aminoschutzgruppen bieten sich abhydrierbare Gruppen wie zum Beispiel der Benzyloxycarbonylrest ( = Z-Rest) oder schwach sauer abspaltbare Gruppen wie zum Beispiel der 2-(p-Diphenyl)-isopropyloxycarbonyl- oder 2-(3,5-Dimethoxyphenyl)-isopropyloxy-carbonylrest an.

Die stufenweise Kondensation erfolgt durch Synthese aus den entsprechenden, gegebenenfalls in üblicher Weise geschützten Aminosauren, in herkömmlicher Weise. Ebenfalls ist die Verwendung automatischer Peptid-Synthesierer, zum Beispiel Beckman Model 990 Peptid Synthetisierer möglich unter Verwendung der auf übliche Weise im Handel erhältlichen geschützten Aminosäuren.

Diese Synthese erfolgt zum Beispiel in der hierfür üblichen Weise aus den entsprechenden geschützten Aminosäuren, indem man zunächst die Carboxy-terminale Aminosäure des zu synthetisierenden Peptids, deren $\alpha$-ständige Aminogruppe geschützt ist, an einen hierfür üblichen synthetischen Träger kovalent bindet, die $\alpha$-Amino-Schutzgruppe abspaltet, an die so erhaltene freie Aminogruppe die nächstfolgende geschützte Aminosäure über ihre Carboxy-Gruppe bindet, dann wiederum die $\alpha$-Amino-Schutzgruppe dieser zweiten Aminosäure abspaltet, hieran die nächste Aminosäure bindet und in dieser Weise Schritt für Schritt die übrigen Aminosäuren des zu synthetisierenden Peptids in der richtigen Reihenfolge verknüpft und nach Verknüpfung aller Aminosäuren das fertige Peptid vom Träger abspaltet und gegebenenfalls weitere vorhandene Seitenfunktions-Schutzgruppen abspaltet.

Die Reaktion zur Verknüpfung der Aminosäuren finden in dem Temperaturbereich von 10 - 40º C, vorzugsweise 20 - 30º C, gegebenenfalls in einem hierfür üblichen indifferenten Lösungs- oder Suspensionsmittel (zum Beispiel Dichlormethan) statt, wobei gegebenfalls zur Verbesserung der Löslichkeit bis zu 20% Dimethylformamid zugesetzt werden kann.

Als synthetisches Trägermaterial kommen synthetische Polymere in Frage, zum Beispiel quellbares Polystyrolharz in Perlenform (beispielsweise ein chlormethyliertes Copolymerisat aus Polystyrol und 1% Divinylbenzol). Als Schutzgruppen für die $\alpha$-ständigen Aminogruppen kommen zum Beispiel in Frage: tertiäre Butyloxycarbonylgruppe, Carbobenzoxygruppe beziehungsweise Carbobenzthiogruppe (gegebenenfalls jeweils mit p-Brom oder p-Nitro-benzylrest). Trifluoracetylgruppe, Phthalylrest, o-Nitrophenoxyacetylgruppe, Tritylgruppe, p-Toluolsulfonylgruppe, Benzylgruppe, im Benzolkern substituierte Benzylreste (p-Brom- oder p-Nitro-benzylrest), $\alpha$-Phenyl-ethylrest. Hierzu wird auch auf das Buch von Jesse P. Greenstein und Milton Winitz, Chemistry of Amino Acids, New York 1961, John Wiley and Sons, Inc., Volume 2, beispielsweise Seite 883 und folgende sowie The Peptids, Volume 2, Ed. E. Gross and J. Meienhofer, Academic from New York, Tabelle III und IV verwiesen. Diese Schutzgruppen kommen grundsätzlich auch für den Schutz von weiteren funktionellen Seitengruppen (OH-Gruppen, $NH_2$-Gruppen) der in Frage kommenden Aminosäuren in Frage.

Vorhandene Hydroxygruppen (Serin, Threonin) werden vorzugsweise durch Benzylgruppen und ähnliche Gruppen geschützt. Weitere nicht $\alpha$-ständige Aminogruppen (zum Beispiel Aminogruppen in w-Stellung; Guanidinogruppe des Arginins) werden vorzugsweise mit Nitro-Gruppen geschützt.

Die Verknüpfung der einzelnen Aminosäuren miteinander erfolgt nach den hierfür üblichen Methoden. Insbesondere kommen in Frage:

Methode der symmetrischen Anhydride (in Gegenwart von Dicyclohexylcarbodiimid), Carbodiimid-Methode,

Carbodiimid-Hydroxybenzotriazol-Methode (siehe The Peptids, Volume 2, Ed. E. Gross and J. Meienhofer).

Für die Verknüpfung von Arginin wird vorzugsweise die Carbodiimid-Methode benutzt, für die Verknüpfung von Asparagin sowie von Glutamin wird vorzugsweise die Carbodiimid-Hydroxy-benzotriazol-Methode benutzt (siehe The Peptids, Volume 2, Ed. E. Gross and J. Meienhofer). Für die übrigen Aminosäuren wird im allgemeinen die Methode der symmetrischen oder gemischten Anhydride benutzt.

Die Herstellung von Peptiden gemäß Anspruch 1, worin jedoch $R^6$ D-Orn oder D-Lys ist (Ausgangsstoffe für die Umsetzung mit Cyanat) kann beispielsweise durch den angegebenen stufenweisen Peptid-Aufbau oder durch Fragmentkondensation erfolgen.

Wenn erfindungsgemäße Peptide hergestellt werden, worin $R^6$ Cit(Q) oder Hci(Q) ist, wird im allgemeinen bei der Peptidsynthese das entsprechende an der endständigen Ureidogruppe durch die Alkylgruppe Q substituierte Cit oder Hci eingesetzt.

Das Überführen der Peptide I in ihre Säureadditionssalze kann durch Umsetzen derselben mit Säuren in an sich bekannter Weise bewerkstelligt werden. Umgekehrt kann das Freisetzen der freien Peptide durch Umsetzen ihrer Säureadditionssalze mit Basen durchgeführt werden.

Bei dem Herstellungsverfahren handelt es sich also zum Beispiel um ausschließliche Festphasentechniken, um teilweise Festphasentechniken, um Fragmentkondensation oder um klassische Lösungsphasensynthese. (Siehe M. Bodanszky, "Principles of Peptide Synthesis", Springer-Verlag, 1984).

Zum Beispiel sind die Methoden der ausschliesslichen Festphasensynthese im Lehrbuch "Solid Phase Peptide Synthesis", J.M. Stewart and J.D. Young, Pierce Chem. Company, Rockford, Ill., 1984 (2. Ausgabe), G. Barany und R.B. Merrifield, "The Peptides", Ch.1, S. 1-285, 1979, Academic Press, Inc. beschrieben.

Klassische Lösungssynthese ist ausführlich in der Behandlung "Methoden der Organischen Chemie (Houben-Weyl). Synthese von Peptiden", E. Wunsch (Herausgeber) (1974), Georg Thieme Verlag, Stuttgart, BRD, beschrieben.

Derartigen Synthesen ist gemein, dass die reaktiven Seitenkettenfunktionsgruppen der verschiedenen Aminosäureresten mit angebrachten Schutzgruppen geschützt werden, die eine chemische Reaktion an dieser Stelle verhindern wird, bis die Gruppe endgültig entnommen ist. Im allgemeinen ist ihnen auch der Schutz einer alpha-Aminogruppe auf einer Aminosäure oder einem Fragment gemein, während das Ganze an der Carboxylgruppe reagiert, worauf die selektive Entnahme der alpha-Aminoschutzgruppen folgt, um die darauffolgende Reaktion an dieser Stelle stattfinden zu lassen. Demgemäss ist es ihnen ebenfalls gemein, ein Zwischenprodukt als eine Stufe der Synthese herzustellen, das jedes der Aminosäurereste umfasst und an seiner gewünschten Sequenz in der Peptidkette angebracht ist, wobei die Seitenkettenschutzgruppen mit den passenden Resten verbunden werden.

Die Verfahren der vorliegenden Erfindung können durchgeführt werden, indem eine Vielzahl von Unterstützungsphasen verwendet werden. Diese Unterstützungsphasen können zum Beispiel Harze wie Benzhydrylaminharze (angebrachterweise 2% netzverbunden), p-Methylbenzhydralaminharze (angebrachterweise 2% netzverbunden) oder ähnliches.

In Formel II:

sind $R^1$, $R^2$ und $R^3$ weiter oben näher beschrieben,

$X_1$ ist Wasserstoff oder eine Acylgruppe, die von geraden oder verzweigten Ketten aliphatischer oder alicyclischer Carbonsäuren mit 1 bis 7 Kohlenstoffatomen abgeleitet sind, oder eine alpha-Aminoschutzgruppe. Die alpha-Aminoschutzgruppen vorgesehen durch $X^1$ sind jene, die aus dem Stand der Technik für die stufenweise Synthese für Polypeptide wohlbekannt sind. Unter den Klassen von alpha-Aminoschutzgruppen, die als $X^1$ verwendet werden können, können Fluorenylmethoxy carbonyl (Fmoc) oder t-Butoxycarbonyl (Boc) genannt werden.

$X^4$ kann eine angebrachte Schutzgruppe für die Hydroxylgruppe des Ser wie zum Beispiel Benzyl (Bzl) und 2,6-Dichlorbenzyl (DCB) sein. Die bevorzugte Schutzgruppe ist Bzl.

$X^5$ kann eine angebrachte Schutzgruppe für die Phenylhydroxylgruppe von Tyr wie zum Beispiel Bzl, 2-Br-Z und 2,6-Dichlorbenzyl (DCB) sein. Die bevorzugte Schutzgruppe ist DCB.

$X^5$ ist eine angebrachte Schutzgruppe für die Seitenkettenaminogruppe von Lys oder Orn. Beispielhaft für angebrachte Seitenkettenaminoschutzgruppen sind Benzyloxycarbonyl (Z) und 2-Chlorbenzyloxycarbonyl [Z-(2-Cl)].

$X^5$ ist eine angebrachte Schutzgruppe für die Guanidinogruppe von Arg, wie zum Beispiel Nitro, Tos, Methyl-(t-Butylbenz)-sulfonyl, 4-Methoxy-2,3,6-trimethylbenzsulfonyl; Tos ist die bevorzugte Gruppe.

$X^{10}$ ist eine die Amidgruppe schützende Benzhydryl- oder Methylbenzhydrylgruppe, die in ein hierfür übliches Tragerharz eingebaut ist (zum Beispiel 98%, Styrol-2%-divinylbenzol Copolymerisat, welches Benzhydrylamin- oder Methylbenzhydrylamingruppen enthält).

Die Wahl einer seitenkettenaminoschutzgruppe ist nicht kritisch, es sei denn, es wird im allgemeinen eine ausgewählt, die im Verlauf der Entfernung der alpha-Aminoschutzgruppen während der Synthese nicht entnommen wird.

Die Peptide der Formel I werden aus Zwischenpeptidharzen der Formel II durch aus dem Stand der Technik bekannte Methoden hergestellt.

Die Festphasensynthese der Zwischenpeptidharze der Formel II wird im wesentlichen wie bei Merrifield, J. Am. Chem. Soc., 85, S. 2149 (1963) beschrieben durchgeführt. Festphasensynthese beginnt am C-Terminal-Ende des Peptids durch Koppeln einer geschützten α-Aminosäure an ein angebrachtes Harz. Derartiges Ausgangsmaterial kann hergestellt werden, indem α-Amino geschütztes Gly oder D-Ala durch eine Amidbindung an einen Benzyhydrylaminharz gebunden wird. Derartige Trägerharze sind allgemein im Handel erhältlich und werden im allgemeinen verwendet, wenn das gewünschte, zu synthetisierende Polypeptid am C-Terminal ein α-Carboxyamid aufweist.

In einer Ausführungsform der Synthese wird die primäre Aminogruppe von Gly oder D-Ala mit einem t-Butoxycarbonylierenden Wirkstoff geschützt und das Koppeln wird durchgeführt, indem man irgendeine der bekannten Dialkylcarbodiimid-Koppel-Verfahren verwendet.

Die Auswahl eines angebrachten Koppelreagens ist dem Fachmann bekannt. Ein besonders angebrachter Koppelreagens ist N,N'-Diisopropylcarbodiimid (DIC).

Aktivierende Reagenzien und ihre Verwendung bei der Peptidkopplung sind in M. Bodanszky, "Principles of Peptide Synthesis", Springer-Verlag, 1984 beschrieben.

Jede geschützte Aminosäure oder Aminosäurensequenz wird in den Festphasenreaktor mit ungefähr doppeltem Ueberschuss eingeführt, und das Koppeln kann in einem Medium von DMF:$CH_2Cl_2$ (1:1) oder in $CH_2Cl_2$ allein durchgeführt werden. In Fällen, bei denen unvollständiges Koppeln auftritt, wird das Koppelverfahren wiederholt, bevor die alpha-Aminoschutzgruppe vor dem Koppeln mit der nächsten Aminosäure entnommen wird. Der Erfolg der Koppelreaktion in jeder Stufe der Synthese wird vorzugsweise durch Ninhydrinreaktion wie bei E. Kaiser, et al., Anal. Biochem., 34, 595 (1970) beschrieben überprüft.

Nachdem die gewünschte Aminosäurensequenz der Peptidharze der Formel II vervollständigt wurde, wird die Boc-Gruppe am Terminal entfernt und, wenn erwünscht, eine N-Terminal-Acylierung durchgeführt, indem ein angebrachtes Acylanhydrid oder Säurechlorid in einem 50-fachen Ueberschuss in einem halogenierten Kohlenwasserstofflösungsmittel verwendet wird; angebrachterweise Essigsäureanhydrid in Methylenchlorid während 30 Minuten. Das Zwischenpeptid kann aus dem Trägerharz entnommen werden, indem es mit einem Reagens wie zum Beispiel flüssiges Wasserstofffluorid behandelt wird, das nicht nur das Peptid vom Harz spaltet, sondern auch alle übrigen Seitenkettenschutzgruppen $X^4$, $X^5$, $X^5$, $X^{10}$ und, falls vorhanden $X^5$ spaltet.

Wird für die Spaltung Wasserstoff-Fluorid verwendet dann sind im Reaktionsröhrchen Anisol oder M-Cresol oder wenn erwünscht Methyläthylsulfid als Spülmittel vorhanden.

Die Überführung von Peptiden II, worin $R^{*6}$ D-Lys oder D-Orn und $X^5$ Wasserstoff ist in Peptide I mittels Cyanat, ist in den vorhergehenden Seiten beschrieben.

Wenn die Acylierung ausgelassen wird, führt die Behandlung von Peptidharze der Formel II mit Wasserstofffluorid zu Dekapeptiden, welche freie omega-Amino und/oder alpha-Aminogruppen aufweisen und Formel II entsprechen, worin $X_1$, $X^4$, $X^5$, $X^5$, $X^{10}$ und, falls vorhanden, $X^5$, Wasserstoff sind. Diese freien Peptide werden in Peptide der Formel I umgewandelt, worin X eine Carbamoylgruppe ist, und zwar durch Behandlung von Cyanat, angebrachterweise Alkalimetallcyanat, vorzugsweise Kaliumcyanat. Die letztgenannce Reaktion, d.h. Umwandlung von $H_2N$ in $H_2N$-CO-NH am Aminoterminus der Peptide und die Umwandlung der Orn/Lys-Reste in die Cit/Hci-Reste kann leicht durch HPLC mittels MeCN-wässrige TFA-Systeme wegen einer charakteristischen 2-3-minutigen Erhöhung der Rückhaltezeiten der carbamoylisierten Peptide beobachtet werden, d.h. Verbindungen mit der $H_2N$-CO-NH-Gruppe, in bezug auf ihre mit der $H_2N$-Gruppe verwandten Verbindungen. Alternativ und bevorzugt werden Peptide I, worin X = Acyl oder Carbamoyl ist direkt aus Zwischenpeptid-Harzen der Formel II, worin $X_1$ Acyl oder Carbamoyl und $R^{*6}$ D-Cit, D-Hci, D-Cit(Q) oder D-Hci(Q) ist, erhalten durch Spaltung und Abspaltung von Schutzgruppen.

Obwohl eine ausschliessliche Festphasensysnthese und eine teilweise Festphasensynthese der Verbindungen der Formel I ebenfalls hierin beschrieben wird, kann die Herstellung der Verbindungen auch mit Hilfe von klassischen Lösungsphasenmethoden hergestellt werden.

Die wie in den Beispielen beschrieben hergestellten, synthetischen Peptide werden mit zwei der potentesten, in letzter Zeit beschriebenen LHRH-Antagonisten verglichen, nämlich [Ac-D-Phe(4-Cl)[1,2], D-Trp[3], D-Arg[6], D-Ala[10]]LHRH (ORG-30276) (Coy, et al., Endocrinology, 100, 1445, 1982) und [Ac-D-Nal(2)[1], D-Phe(4-F)[2], D-Trp[3], D-Arg[6]]LHRH (ORF 18260) (Rivier, et al., In: Vickery, B.H., Nestor, Jr., J.J. Hafez, E.S.E. (Eds.), "LHRH and Its Analogs", S. 11-22, MTP Press, Lancaster, UK, 1984), und es wurde festgestellt, dass sie ähnlich hohe Hemmwirkungen sowohl in vitro als auch in vivo aufwiesen, allerdings mit der Ausnahme, dass sie im Gegensatz zu den Kontrollpeptiden keine in vivo oedematöse Wirkungen zeigten.

Die hormonale Wirksamkeiten in vitro wurden verglichen mit superfundierten Zellsystemen der Hirnanhangsdrüse von Ratten (S. Vigh and A.V. Schally, Peptides. 5 suppl. 1: 241-247, 1984) bei denen die Wirksamkeit von LHRH (und anderen freisetzenden Hormonen) kann genau festgestellt werden, da die Menge des LH (oder anderer Hirnanhangsdrüsenhormone), die in das ausströmende Medium abgegeben wird, nicht nur proportional zur Hormon freisetzenden Potenz der angewandten Peptide ist, sondern ebenfalls leicht messbar anhand von gut beschriebenen Radioimmunotests ist.

Um die Potenz eines LHRH-Antagonisten zu bestimmen, werden Mischungen, die LHRH in einer gleichbleibenden Konzentration (herkömmlicherweise 1 nM) und den Antagonisten in wechselnden Konzentrationen enthalten, für das Superfundieren verwendet, um das Molekularverhältnis des Antagonisten zum LHRH zu bestimmen, bei dem das Wirken des LHRH vollständig blockiert ist. Diese Verhältnisse sind ungefähr 5 für beide Peptide der vorliegenden Erfindung und die Vergleichspeptide, wenn das Zellsystem der Hirnanhangsdrüse der Ratte zuvor mit Antagonisten während 9 Minuten vorinkubiert wird.

7

Bei einem in vivo Ovulationshemmungstest (A. Corbin and C.W. Beattie; Endocr. Res. Commun. 2, 1-23, 1975; D.H. Coy, et al., Endocrinology, 100, 1445, 1982) wurden bei den erfindungsgemässen Peptiden ebenfalls festgestellt, dass sie ungefähr gleich wirksam sind, wie der Kontrollantagonist, nämlich 87,5 - 100% Hemmung der Ovulation kann bei einer subkutan verabreichten Dosis von 1-3 $\mu$g/Ratte für jedes Peptid beobachtet werden.

Beim oedematischen Versuch von Schmidt, et al. (Contraception, 29, 283-289, 1984) konnte jedoch ein ausgeprägter Unterschied zwischen den Kontrollpeptiden und den erfindungsgemässen Peptiden festgestellt werden. Die Kontrollpeptide bewirken Oedeme im Gesicht und auf den Extremitäten, wenn diese Ratten subkutan in Dosen von 1,25 oder 1,5 mg/kg verabreicht wurden. Bei den erfindungsgemässen Peptiden konnten keine derartige Reaktion beobachtet werden, wenn sie subkutan in einer Dosis von 1,5 mg/kg verabreicht wurden.

In den durchgeführten Versuchen wurden die Ratten in drei Gruppen zu je fünf Ratten pro Gruppe und pro untersuchter Verbindung eingeteilt. Ein Vergleich wurde angestellt mit einer bekannten Verbindung aus dem Stand der Technik mit der Bezeichnung ORG 30276 (N-Ac-D-p-Cl-Phe$^{1,2}$,D-Trp$^3$, D-Arg$^6$, D-Ala$^{10}$).

Einmal pro Tag an zwei aufeinanderfolgenden Tagen wurde den Gruppen subkutan LHRH-Antagonisten mit einer Dosierung von 1,5 mg/kg injiziert. Einer Kontrollgruppe wurde nur Verdünnungsmittel injiziert. Die Ratten wurden während 5 Stunden pro Tag beobachtet. Die Reaktionen der Ratten wurden wie folgt klassifiziert: NR keine sichtbare Reaktion, PR teilweises Ansprechen: Oedeme im nasal und paranasalen Bereich, FR vollständiges Ansprechen: Oedem im Gesicht mit oedematösen Extremitäten.

Die Ergebenisse sind in Tabelle 1 unten zusammengefasst.

Tabelle 1

| LHRH Antagonist | 1. Tag | | | 2. Tag | | |
|---|---|---|---|---|---|---|
| | NR | PR | FR | NR | PR | FR |
| ORG 30276 | 3 | 7 | 0 | 0 | 0 | 10 |
| Kontrolle | 9 | 0 | 0 | 9 | 0 | 0 |
| Beispiel I | 8 | 0 | 0 | 8 | 0* | 0 |
| Beispiel III | 8 | 0 | 0 | 8 | 0 | 0 |
| Beispiel IV | 9 | 0 | 0 | 9 | 0 | 0 |
| Beispiel V | 9 | 0 | 0 | 8 | 1* | 0 |
| Beispiel XX | 9 | 0 | 0 | 8 | 1* | 0 |
| Beispiel XXI | 9 | 0 | 0 | 9 | 0 | 0 |
| Beispiel XXVI | 8 | 0 | 0 | 8 | 0 | 0 |
| Beispiel XXVII | 9 | 0 | 0 | 9 | 0 | 0 |

* sehr geringes Gesichtsoedem

Alle dargestellten Peptide sind vollständig wirksam bei der Hemmung der LHRH-Ausschüttung in einigermassen vernünftigen Konzentrationen in vitro, obwohl die meisten etwas weniger potent sind als die vorliegenden in vitro-Standards; allerdings sind diese Peptide in vivo viel potenter.

Dies wurde bei einem Versuch über die Histamin-Freisetzung in vitro aus den Bauchfell-Mastzellen gezeigt, der in Uebereinstimmung mit dem Verfahren nach Morgan et al. (Int. Archs. Allergy appl. Immun. 80, 70, 1986) durchgeführt wurde.

In vitro Freisetzung von Histamin

Bei diesem Versuch wurden Ratten mit Aether narkotisiert und Bauchhöhlenexsudatzellen wurden geerntet, indem sie mit 12 ml Mastzellenmedium (MCM) (150 m M NaCl; 3 m M KCl; 3,0 m M Na$_2$HPO$_4$; 3,5 m M KH$_2$PO$_4$, 0,98 m M CaCl; 5,6 m M Dextrose, 0,1%iges Kalbsserumalbumin; 0,1%ige Gelatine und 10 E/ml Heparin) [9] gewaschen wurden. Zellen von 4 oder 5 Ratten wurden gesammelt, bei 120 G zentrifugiert, mit MCM auf eine Konzentration von 0,5 x 10$^6$ ml resuspendiert und 1 ml wurde in 12 x 75 mm Polyäthylen-Röhrchen aliquotiert. Die Röhrchen wurden während 15 min. auf 37ºC ausgeglichen und allein (Hintergrundfreisetzung von Histamin) mit 48/80 (positive Kontrolle) (Sigma Chemicals, St. Louis, Mo.) oder mit angebrachten Konzentrationen (1 ng bis 10 ug/ml) an LHRH-Antagonisten während 60 min. inkubiert. Die Reaktion wurde durch Abkühlen der Röhrchen auf 4ºCbeendet. Die Röhrchen wurden zentrifugiert; die Ueberstände wurden entfernt und bei -20ºC gelagert, bis sie nach Histamin untersucht wurden. Die Versuche wurden doppelt durchgeführt. Das gesamte Zellhistamin wurde durch Sieden während 10 min. festgestellt. Das in Reaktion auf den Antagonisten ausgeschüttete Histamin wurde als Prozentsatz der gesamten Ausschüttung ausgedrückt. Diese Konzentrationen, die in ungefähr 50% des gesamten Mastzellen-Histamins (HRD$_{50}$ $\mu$g/ml) ausgeschüttet wurde, wurde für jeden Antagonisten bestimmmt. Die Ergebnisse wurden in Figur 1 zusammengefasst.

Bei allen Peptiden wurde eine Wirksamkeit bei der Hemmung der Ovulation bei weiblichen Säugetieren bei sehr niedrigen Dosen festgestellt. Die Peptide der vorliegenden Erfindung wurden oft in Form von pharmazeutisch annehmbaren, nicht toxischen Salzen, wie zum Beispiel Säureadditionssalzen, verabreicht. Beispiele für derartige Säureadditionssalze sind Hydrochloride, Hydrobromide, Sulfat, Phosphat, Fumarat, Glukonat, Tannat, Maleat, Acetat, Citrat, Benzonat, Succinat, Alginat, Pamoat, Malat, Ascorbat, Tartrat und ähnliche. Wenn der Wirkstoff in Tablettenform verabreicht wird, kann die Tablette ein pharmazeutisch annehmbares Verdünnungsmittel, das ein Bindemittel wie zum Beispiel Tragant, Maisstärke oder Gelatine umfasst, enthalten; Zerfallmittel wie Alginsäure und ein Gleitmittel wie Magnesiumstearat.

Wenn Verabreichung in flüssiger Form erwünscht wird, kann ein Süssmittel und/oder Geschmackstoff als Teil des pharmazeutisch annehmbaren Verdünnungsmittels verwendet werden, und bei intravenöser Verabreichung wird diese in isotonischer Saline, Phosphatbufferlösungen oder ähnlichem durchgeführt.

Die pharmazeutischen Zusammensetzungen werden herkömmlicherweise das Peptid in Zusammenhang mit einem üblichen, pharmazeutisch annehmbaren Träger enthalten. Ueblicherweise wird die Dosis von ungefähr 1 bis 100 Mikrogramm des Peptids pro Kilogramm Körpergewicht des Wirts bei intravenöser Verabreichung betragen; die oralen Dosen werden höher sein. Im allgemeinen wird die Behandlung von Versuchspersonen mit diesen Peptiden auf die gleiche Art und Weise durchgeführt wie die klinische Behandlung mit anderen LHRH-Antagonisten.

Diese Peptide können Säugetieren intravenös, subkutan, intramuskulär, oral, intranasal oder intravaginal verabreicht werden, um die Fruchtbarkeitshemmung und/oder -Kontrolle zu erreichen und ebenfalls bei Anwendungen, die einer reversiblen Unterdrückung der gonadalen Aktivität bedürfen, wie zum Beispiel bei der Behandlung von vorzeitiger Pubertät oder während Bestrahlungs- oder Chemotherapie. Wirksame Dosen variieren je nach Verabreichungsform und den jeweiligen zu behandelnden Säugetierarten. Ein Beispiel einer typischen Dosierungsform ist eine physiologische Salinenlösung mit Peptid, die in einer Dosis von ungefähr 0,1 bis 2,5 mg/kg Körpergewicht verabreicht wird. Die orale Verabreichung des Peptids kann entweder in fester oder flüssiger Form durchgeführt werden.

Obwohl die Erfindung im Hinblick auf die bevorzugte Ausführungsform beschrieben worden ist, sollte es selbstverständlich sein, dass Wechsel und Abänderungen, die für den Fachmann offensichtlich sind, gemacht werden können, ohne dass der durch die beiliegenden Patentansprüche begrenzten Erfindungsbereich verlassen wird. Substitutionen aus dem Stand der Technik, die nicht wesentlich von der Wirksamkeit abweichen, können ebenfalls in der Erfindung verwendet werden.

SB-030, SB-075 und SB-088 sind Dekapeptide wie unten näher beschrieben, die besonders vorteilhafte Wirksamkeiten aufweisen:

Ac-D-Nal(2)-D-Phe(4Cl)-R$^3$-Ser-Tyr-R$^6$-Leu-Arg-Pro-D-Ala-NH$_2$

SB-030, R$^3$ = D-Trp, R$^6$ = D-Cit;

SB-075, R$^3$ = D-Pal, R$^6$ = D-Cit;

SB-088, R$^3$ = D-Pal, R$^6$ = D-Hci.

Von den enthaltenen Aminosäuren der Bestandteile sind D-Pal, D-Cit und D-Hci nicht im Handel erhältlich. Für D-Pal wird eine völlige Synthese benötigt, die mit Diäthylacetamidomalonat und 3-Picolylchlorid beginnt und auf bekannte Weise durchgeführt wird. D-Cit und D-Hci können leicht aus im Handel erhältlichen Diaminosäuren, wie zum Beispiel D-Orn bzw. D-Lys, hergestellt werden.

Jedes Peptid weist eine hohe LHRH-Hemmwirksamkeit wie unten gezeigt auf und ist völlig frei von oedematogenischen Nebenwirkungen.

Tabelle 1. LHRH-Hemmwirksamkeiten von SB-030, SB-075
und SB-088

| Peptid | % Hemmung von LHRH (3nM) mit equimolärem Peptid | | Ovulationshemmungsversuch | |
|---|---|---|---|---|
| | bei 0 min. | nach 2 Std. | Dosis (ug) | %Hemmung |
| SB-030 | 83 | 58 | 1,5 | 50 |
| | | | 3,0 | 100 |
| SB-088 | 86 | 60 | 1,5 | 66 |
| | | | 2,0 | 83 |
| | | | 3,0 | 100 |
| SB-075 | 89 | 54 | 1,5 | 75 |
| | | | 2,0 | 100 |

Abkürzungen: Die Aminosäuren und Schutzgruppen werden wie von der IUPAC IUB JCBN [Eur. J. Biochem.138, 9-37 (1984)] empfohlen abgekürzt. Andere Abkürzungen sind: $Boc_2O$, di-t-Butyldicarbonat; DCM, Dichlormethan; TLC/TLE, Dünnschichtchromatographie/Elektrophorese auf Kieselgel-Platte.

SB-030 weist gegenüber SB-075 und SB-088 den Vorteil auf, dass es nur eine ungewöhnliche Aminosäure, nämlich D-Cit, umfasst. Andererseits weist sowohl SB-075 als auch SB-088 eine höhere Potenz und bessere Wasserlöslichkeit als SB-030 auf.

Die Beobachtungen und Versuche, die während der Synthese von SB-030, SB-075 und SB-088 gemacht worden sind, können wie folgt zusammengefasst werden:

Die Festphasensynthese von SB-030, SB-075 und SB-088 auf einem Benzhydrylaminharz unter Verwendung des Boc-Protokolls wird beschrieben. Dieses Vorgehen ist für die Ansammlung dieser Peptide völlig ausreichend, da es die schrittweise Kettenverlängerung durch das Carboxylterminal beinhaltet, um bedeutende Racemisierung während der Amidbindungsbildung zu vermeiden und ebenfalls die leichte Isolierung der Zwischenprodukte, die sich als besonders schwierig erweisen können während der Synthese von Fragmenten mit hydrophilischem Charakter (d.h. Fragmente 9-10 bis 5-10).

Die herkömmliche, stufenweise Synthese in einer Lösung ist natürlich auch eine angebrachte Methode zur Herstellung dieser Peptide, jedoch ist jenes Verfahren sicher arbeits- und zeitaufwendiger.

AUSGANGSMATERIALIEN

Feststoffträger

Ein Benzhydrylaminharz mit 1,1 mAeq $NH_2$/g (Advanced ChemTech, Louisville, KY) wurde verwendet. Harze mit niedrigerem $NH_2$-Gehalt, z.B. 0,4-0,6 mAeq/g, können ebenfalls verwendet werden, sind aber natürlich weniger wirtschaftlich im Verbrauch.

Aminosäurenderivate

Die unten aufgeführten Boc-Aminosäuren wurden bei Bachem erstanden und ohne weitere Reinigung verwendet:

Boc-D-Ala, Boc-Arg(Tos), Boc-Leu.$H_2O$,
Boc-D-Nal, Boc-D-Phe(4Cl), Boc-Pro,
Boc-Ser(Bzl), Boc-D-Trp und Boc-Tyr(DCB).

Boc-D-Cit, Boc-D-Hci und Boc-D-Pal, die nicht im Handel erhältlich sind, wurden gemäss der Literatur (1-4) mit geringfügigen Aenderungen hergestellt.

Boc-D-Cit

Stufe 1, D-Cit

D-Orn (Sigma, 98%) wurde in D-Cit wie beschrieben (1) umgewandelt, mit Ausnahme davon, dass die Reaktionsmischung von $(D-Orn)_2Cu$ und KOCN während 2 bis 3 Stunden gerührt wurde, um die Ablagerung einer Kruste aus (D-Cit, D-Orn)Cu auf der Behälterwand zu verhindern.

Die Reinheit des D-Cit wurde anhand von TLE (Dünnschichtelektrophorese) bei eine pH-Wert von 6,5 Pyridin-Essigsäure-Wasser (100:4:900) Puffer und bei 30 V/cm während 45 min. geprüft.

Stufe 2, Boc-D-Cit

6,6 g (30 mMol) $Boc_2O$ in 15 ml Acetonitril wurde einer Lösung von 4,4 g (25 mMol) D-Cit in 25 ml Wasser und 12,6 ml (90 mMol) Triäthylamin beigefügt. Die Mischung wurde während 2 bis 3 Stunden gerührt. Danach wurde die Mischung einer TLC (Dünnschichtchromatographie) unterworfen, indem ein Lösungsmittelsystem aus Aethylacetat-Pyridin-Essigsäure-Wasser (60:10:3:6) verwendet wurde: Boc-D-Cit, D-Cit und Boc-D-Orn (Boc), die aus der D-Orn-Verunreinigung gebildet wurde, weisen RFs von jeweils 0,4, 0,0 und 0,9 auf. Die Mischung wurde verdünnt mit 25 ml Wasser und extrahiert mit mit Diäthyläther (2x20 ml) und Aethylacetat (2x20 ml), um den Ueberschuss an Dicarbonat und Boc-D-Orn(Boc)-Verunreinigung zu entfernen. Die wässrige Phase wurde mit 1 M $KHSO_4$ gesäuert und nach Beigabe von ungefähr 10 g $Na_2SO_4$ wurde die Phase mit Aethylacetat (3x30 ml) extrahiert. Kombinierte Aethylacetatlösungen (sorgfältig von der wässrigen Schicht getrennt) wurden unter Vakuum verdampft. Der Rest wurde in trockenem Dioxan verdünnt und dreimal unter Vakuum verdampft. Das dadurch gebildete leichte Oel wurde in 25 ml DMF verdünnt und zum Koppeln als mMol/ml dieser Grundlösung aus Boc-D-Cit verwendet.

Die Synthese des Boc-Cit unter Verwendung von Boc-Azid als Acylierungswirkstoff und die Herstellung des kristallinen Dicyclohexylaminsalzes wurden beschrieben (2).

Boc-D-Hci

Stufe 1, D-Hci

D-Hci wurde aus D-Lys.HCl (Sigma) in Analogie zu D-Cit (siehe oben und Verweis 1) hergestellt.

Stufe 2, Boc-D-Hci

6,6 g Di-tert-Butyldicarbonat verdünnt in 15 ml Acetonitril wurde einer Lösung aus 25 mMol (4,73 g) D-Hci in 25 ml Wasser und 12,6 ml (90 mMol) Triäthylamin beigefügt, und die Reaktionsmischung wurde während 2 bis 3 Stunden gerührt. Die Vollständigkeit der Reaktion wurde anhand von TLC wie im Falle von Boc-D-Cit geprüft; die RFs für Boc-D-Hci, D-Lys und Boc-D-Lys(Boc) betrugen jeweils 0,3, 0,0 und 0,8. Die Reaktionsmischung wurde mit Aether extrahiert. Die wässrige Phase wurde mit 1 M $KHSO_4$ gesäuert, und nachdem 10 g $Na_2SO_4$ beigefügt wurde, wurde sie mit einer 1:1-Mischung aus Aethylacetat und n-Butanol (4x30 ml) extrahiert. Die kombinierten, organischen Lösungen wurden über $Na_2SO_4$ getrocknet und unter Vakuum verdampft. Der Rest wurde mit Diäthyläther trituriert, filtriert, mit Aether gewaschen, dann in Aethylacetat suspendiert und während 2 Stunden bei 20°C gerührt und bei 0°C nochmals für 2 weitere Stunden gerührt. Das so erhaltene, kristalline Material wurde abfiltriert, mit kaltem Aethylacetat gewaschen und getrocknet, um ungefähr 3,9 g Boc-D-Hci mit einem Schmelzpunkt von 141-142°C (dec.) zu bilden.

Boc-D-Pal(3)

Stufe 1, Diäthylacetamido(3-pyridylmethyl)malonat (3) 113,0 g (60,3%) Diäthylacetamido(3-pyridylmethyl)malonat wurden aus 132,0 g Diäthylacetamidomalonat mit Hilfe des Verfahrens nach J.E. Rivier et al. (3) erhalten. Das rohe Material schmilzte bei 95°C. Eine kleine Probe wurde aus bei 104-106°C geschmolzenenWasser (Literatur 95°C) rekristalliziert.

Stufe 2, N-Acetyl-3-(3-pyridyl)-DL-Alaninäthylester (4) 61,6 g (200 mMol) Diäthylacetamido(3-pyridylmethyl)malonat wurden in 210 ml ethanolischem NaOH in einer mit einem Stopfen versehenen Flasche verdünnt und über Nacht bei Raumtemperatur stehen gelassen. Am nächsten Tag wurde die Mischung unter Rückfluss gesetzt und auf dieser Temperatur während 30 min. gehalten. Die Mischung wurde dann auf Raumtemperatur abgekühlt und filtriert, um die unlösslichen Feststoffe zu entfernen. Die unlösslichen

Stoffe wurden mit verschiedenen kleinen Teilen von Aethanol gewaschen und dann weggeschüttet. Die kombinierten Filtrate wurden bis zur Trockenheit verdampft und der trockene Rest aus Wasser kristallisiert, indem 3-5 ml $H_2O$ pro Gramm Feststoff verwendet wurden. 38,0 g (160 mMol, 80%) Wiedergewinn des Materials. Schmelzpunkt 124-126°C (Literatur 118-121°C).

Stufe 3, N-Acetyl-3-(3-pyridyl)-D-Alaninäthylester (3) 45,0 g (190 mMol) der obenbeschriebenen DL-Mischung wurden in 225 ml heissem 4,5%igen HCl verdünnt. Diese Lösung wurden dann schnell auf 25°C abgekühlt, um eine Suspension aus feinen Kristallen zu bilden. Der pH-Wert wurde auf 7,2 gebracht, indem 2 N KOH verwendet wurden und 150 mg alpha-Chymotrypsin dann beigefügt wurden, und der pH-Wert wurde dann bei 6,8-7,2 gehalten, indem tropfenweise 2 N KOH beigefügt wurden. Nachdem sich keine Aenderung im pH-Wert während 15 min. gezeigt hat, wurden weitere 50 mg alpha-Chymotrypsin beigefügt, um sicher zu stellen, dass die Hydrolyse vollständig gelaufen ist. Es zeigte sich kein weitere Wechsel im pH-Wert, nach dieser frischen Beigabe des Enzyms und der endgültige pH-Wert betrug 7,2. Diese Lösung wird mit 4x200 ml Aethylacetat extrahiert. Die Aethylacetat-Entzüge werden kombiniert, über $Na_2SO_4$ gerocknet, Trockenmittel ausfiltriert und das Lösungsmittel unter Vakuum entfernt. Der Rest wird aus Toluol (13-15 ml/g) rekristallisiert, um einen Wiedergewinn von 13,0 g (55 mMol, 57%) mit einem Schmelzpunkt von 79-81°C (Literatur 122°C) zu bilden.

Stufe 4, 3-(3-Pyridyl)-D-Alanin, D-Pal(3) (4) 13,0 g (55 mMol) N-Acetyl-3-(3-pyridyl)-D-Alaninäthylester werden über Nacht mit 100 ml 6 N HCl unter Rückfluss gesetzt. Das Lösungsmittel wird unter Vakuum entfernt. 15 mg Aethanol wurden beigefügt und dieses wurde unter Vacuum entfernt. Diese Aethanolbehandlung wird zwei weitere Male wiederholt. Der Rest wird aus 90%igem Aethanol rekristallisiert, um 13,0 g (54 mMol, 98%) Amindihydrochlorid zu ergeben. Schmelzpunkt 237-239°C (dec.) (Literatur 253-256°C).

Stufe 5, N-Boc-3-(3-pyridyl)-D-Alanin, Boc-D-Pal(3) (3) 13,0 g (54 mMol) des oben beschriebenen D-Pal(3) wurden in 50 ml $H_2O$ verdünnt. 50 ml Tetrahydrofuran und 30 ml (214 mMol) Triäthylamin wurden beigefügt. Dieser Mischung wurde während des Rührens in stündlichen Abschnitten 4x5 g-Teile $Boc_2O$ (90,7 mMol) beigefügt. Nach dem Rühren während im ganzen 6 Stunden wird das Tetrahydrofuran unter Vakuum freigelegt. Ueberschüssiges Dicarbonat wird mit Hexan entfernt. Die wässrige Schicht wird mit 6 N HCl auf eine pH-Wert von 4 gebracht und dann mit 4x100 ml-Teilen Aethylacetat extrahiert. Das Aethylacetat wird kombiniert und 2x5 ml Teile der gesättigten Salzlösung rückextrahiert. Ueber $Na_2SO_4$ wird getrocknet, gefiltert und Lösungsmittel unter Vakuum freigelegt. Rest wird aus Toluol rekristallisiert, um 8,4 g (31 mMol, 57%) Boc-D-Pal(3) mit einem Schmelzpunkt von 141-142°C (dec.) (Literatur 135-136°C) zu ergeben.

HERSTELLUNG DES PEPTIDHARZES

Ein Reaktionsgefäss für manuelle Synthese von ungefähr 60 ml Fassungsvermögen wurde mit 1 g (1,1 mMol) Benzhydrylamin.HCl Harz gefüllt. Man liess während 2 Stunden in 20 ml DCM anschwellen und filterte dann. Danach wurden 15 ml 10%iges DIEA/DCM dem Harz beigefügt, während 2 min. geschüttelt und filtriert. Letztere Behandlung wurde zwei weitere Male wiederholt. Die Boc-Aminosäuren als vorgeformte HOBt-Ester wurden wie in Tabelle 2 dargestellt nach und nach an diesen Harz gekoppelt. Stufen 1 - 12 bilden eine Kopplungszyklus für einen Aminosäurenrückstand. Zehn nacheinanderfolgende Zyklen würden einen Dekapeptidamidharz mit freiem D-Nal(2)-Rest terminiert ergeben. Dieser Peptidharz wurde acetylisiert wie in Stufen 2e und 2f beschrieben, dann aus dem Reaktionsgefäss entnommen, mit DCM und n-Hexan gewaschen und unter Vakuum getrocknet, um 2,9-3,0 g des gewünschten Acetyl-Dekapeptidamid-Harzes[2] mit den folgenden Strukturen zu bilden:

1, Ac-d-Nal(2)-D-Phe(4Cl)-D-Trp-Ser(Bzl)-Tyr(DCB)-D-Cit-Leu-Arg(Tos)-Pro-D-Ala-NH-HARZ, für SB-030;

2, Ac-D-Nal(2)-D-Phe(4Cl)-D-Pal(3)-Ser(Bzl)-Tyr(DCB)-D-Cit-Leu-Arg(Tos)-Pro-D-Ala-NH-HARZ, für SB-075;

3, Ac-D-Nal(2)-D-Phe(4Cl)-D-Pal(3)-Ser-(Bzl)-Tyr(DCB)-D-Hci-Leu-Arg(Tos)-Pro-D-Ala-NH-HARZ, für SB-088.

[2]SB-088 wurde in einer kleineren Menge (0,1866 mMol) synthetisiert, das Peptidharz Nr. 3 und das rohe HF-Salz des erhaltenen freien Dekapeptids betrugen jeweils 410 mg und 200 mg.

Tabelle 2. Syntheseprogramm

| STUFE | REAGENS UND VERFAHREN | MISCH-ZEITEN IN MIN |
|---|---|---|
| 1 | vorbereiteter HOBt-Ester[3], Koppeln | 90 |
| 2 | Ninhydrin-Test $(5)^4$, wenn die Kügelchen - mittelmässig oder stark blau sind werden Stufen 1 + 2 wiederholt, bei leicht blauer Färbung mit Stufen 2a-2f weiterfahren | |
| 2a | MeOH (10 ml), waschen | 1 |
| 2b | 10%iges DIEA/DCM (10 ml), Neutralization | 2 |
| 2c | MeOH (10 ml), waschen | 1 |
| 2d | DCM (10 ml), (dreimal) waschen | 2 |
| 2e | Acetylimidazol[4], Acetylisierung | 30 |
| 2f | Ninhydrin-Test, wenn positiv, Stufen 2d - 2f wiederholen | |
| 3 | MeOH (10 ml), (zweimal) waschen | 1 |
| 4 | DCM (10 ml), (dreimal) waschen | 2 |
| 5 | 50%iges TFA/DM (10 ml), entblocken | 1 |
| 6 | 50%iges TFA/DM (10 ml), entblocken | 30 |
| 7 | IpOH (10 ml), waschen | 1 |
| 8 | 10%iges DIEA/DCM (10 ml), Neutralization | 3 |
| 9 | MeOH (10 ml), waschen | 1 |
| 10 | 10%iges DIEA/DCM (10 ml), Neutralization | 3 |
| 11 | MeOH (10 ml), waschen | 1 |
| 12 | DCM (10 ml), waschen | 2 |

[3]Mit Ausnahme von Boc-D-Cit, Boc-Aminosäure-HOBT-Estern wurden wie folgt hergestellt:

3 mMol Boc-Aminosäure [ d.h. (in mg) D-Ala:568, D-Pro:646, Arg(Tos):1286, Leu:748, Tyr(DCB):1321, Ser(Bzl):886, D-Hci-865, D-Trp:913 ODER D-Pal(3):799, D-Phe(4Cl):899, D-Nal(2):946] und 460 mg (3 mMol) HOBT.$H_2O$ wurden in 1 ml DMF gelöst, mit 2 ml DCM verdünnt, auf $0^{\circ}C$ abgekühlt und mit 0,6 ml (3,8 mMol) DIC bei $0^{\circ}C$ während 10 min. zum Reagieren gebracht; danach dem Harz mit 4x1 ml DCM-Spülung beigefügt. Für Boc-D-Cit wurde HOBt.$H_2O$ in 3 ml Grundlösung aus Boc-D-Cit gelöst, mit Dic wie oben zum Reagieren gebracht, dann dem Harz mit 1x1 ml DMF und 3x1 ml DCM-Spülung beigemischt.

[4]Im Falle von Cit-enthaltenden Peptidharzen fällt der Test negativ aus, wenn die Kügelchen weiss sind (oder schwach gelb) und die Lösung lila gefärbt sind. Das vollständige Koppeln des Boc-Arg(Tos) an den Pro-Rest auf dem Harz kann geprüft werden, indem eine Isatinreagens (2,5 g Isatin, 20 g Phenol, 30 g Benzylalkohol und 2,5 g Boc-Phe) verwendet wird; der Test fällt negativ aus, wenn die Kügelchen weiss sind (oder leicht gelb **aber nicht blau**) nach 3 bis 4-maligem Waschen mit Aceton.

[4]0,34 g Imidazol gelöst in 2 ml DCM wurde mit 0,5 ml acetischem Anhydrid bei $0^{\circ}C$ während 2 min. zum Reagieren gebracht und dann dem Harz mit 3x2 ml DCM-Spülung beigefügt.

14

HERSTELLUNG DER FREIEN DEKAPEPTIDAMIDE

Spaltung und Entschützung

Jeder Peptidharz wurde mit 3 ml M-Cresol und ungefähr 30 ml Wasserstofffluorid (HF) bei 0°C während 45 min. behandelt. Nach der Entfernung des HF unter Hochvakuum, wurde der übrige Peptidharz mit trockenem Diäthyläther trituriert, filtriert, mit Diäthyläther gewaschen und das Peptid wurde dann mit folgendem extrahiert:

1, 50%ige, Essigsäure und DMF (2-2 x 20 ml) für SB-030;

2, 50%ige, Essigsäure (3 x 15 ml) für SB-075 und SB-088;

und aus dem Harz durch Filtrieren getrennt. Die Peptidlösung wurde unter Vakuum verdampft und der Rest wurde mit Diäthyläther trituriert, filtriert, mit Diäthyläther gewaschen und getrocknet, um 1,2-1,3 g HF-Salz mit rohen Dekapeptiden[2] mit einer Reinheit von 70-85% zu bilden.

Reinigung

Das rohe Peptid wurde gereinigt, indem ein Beckman Prep-350 präparatives HPLC-System verwendet wurde. Die Trennungen wurden auf einer 41,5x250mm Dynamax-Säule erreicht, die mit sphärischem $C_{18}$ Kieselgel gepackt war (300 A Porengrösse, 12 $\mu$m Teilchengrösse) (RAININ Inc. Co. Woburn, MA). Rohes SB-030 wurde auf der Säule in einer 30%igen, Ameisensäure Lösung angewandt, während rohes SB-075 und SB-088 in 30%iger, Essigsäure gelöst wurden. Druckgefälleelution wurde verwendet. Lösungsmittel A enthielt 0,1%iges, wässrige TFA und Lösungsmittel B war 0,1%iges TFA in 70%igem, wässrigem Acetonitril. Die Druckflussgeschwindigkeit betrug 35 ml/min. Die Säuleneluierung wurde anhand eines variablen Wellenlängen-UV-Detektors (Beckman Typ 163) bei 220 nm überwacht.

Der Betrag der reinen Dekapeptidamide und die allgemein erhaltenen Erträge waren die folgenden:

1, 600 mg (34%) SB-030,

2, 960 mg (52%) SB-075 und

3, 140 mg (45%) SB-088 (siehe Fussnote 2),

mit einer Reinheit von >95%.

Analytisches HPLC

Die HPLC-Analyse wurde mit einem Hewlett-Packard Modell 1090 Flüssigchromatograph durchgeführt. Die Peptidproben wurden auf eine 4,6x250 mm W-Porex 5 um, $C_{18}$-Säule (Phenomex, Rancho Palos Verdes, CA) mit einer Durchflussgeschwindigkeit von 1,2 ml/min. unter Verwendung von Druckgefälleelution mit den Lösungsmitteln A und B chromatographiert (Chromatogramme der rohen Proben liegen bei).

Aminosäurenanalyse

Die Peptidproben wurden bei 110°C während 20 Stunden in evakuierten, versiegelten Röhrchen unter Verwendung von 4 M methansulfonischer Säure mit 0,2% 3-(2-Aminoäthyl)indol hyrolisiert, und die Aminosäurenzusammensetzung des Hydrolyzats wurde in einem Beckman 6300 Aminosäurensanalysator bestimmt.

Die beiliegende Tabelle zeigt die Affinität der LHRH-Analoge für Hirnanhangsdrüse oder Rezeptoren der menschlichen Brustkrebsmembrane, wie bestimmt unter Verwendung von radioaktivmarkiertem LHRH oder radioaktivmarkiertem D-Trp[6] LHRH.

Die Messungen mit radioaktivmarkiertem LHRH (A) zeigen, dass SB-075 die höchste Affinität für die Hirnanhangsdrüsenrezeptoren aufweist, d.h. die grösste Fähigkeit hat, LHRH zu verschieben, was eines der Hauptmerkmale für LHRH-Antagonisten ist. Andererseits scheint SB-088 eine besonders hohe Affinität für Rezeptoren der menschlichen Brustkrebsmembrane in beiden Versuchen (A + B) zu zeigen.

LITERATURVERWEIS

1 J.P. Greenstein and M. Winitz: Chemistry of the Amino Acids, John Wiley + Sons, New York, NY, 1961. (Cit: 2491 - 2494)

2 K. Eisele: Hoppe-Seyler's Z. Physiol. Chem. 356, 845-854 (1975).

3 J.E. Rivier, J. Porter, C.L. Rivier, M Perrin, A. Corrigan, W.A. Hook, R.P. Siraganian and W.W. Vale: J. Med. Chem. 29, 1846-1851 (1986).

4 C. Hoes, J. Raap, W. Bloemhoff and K.E.T. Kerling: Recl. Trav. Chim. Pay-Bas 99, 99-104 (1980).
5 J.M. Stewart and J.D. Young: Solid Phase Peptide Synthesis, Second Edition. Pierce Chemical Company, Rockford Illionois, 1984[6].

BEISPIEL I

Die Synthese des Peptids der Formel

AC-D-Nal(2)-D-Phe(4-Cl)-D-Trp-Ser-Tyr-D-Hci-Leu-Arg-Pro-D-Ala-NH$_2$ erfolgt aus dem Zwischenpeptid Ac-D-Nal(2)-Pro-D-Phe(4-Cl)-D-Trp-Ser-Tyr-D-Lys-Leu-Arg-Pro-D-Ala-NH$_2$. Dieses Zwischenpeptid wurde Schrittweise auf einem ca. 0,6 mAeq NH$_2$/g enthaltendem Benzhydrylaminharz auf einem Beckman 990 Synthesizer aufgebaut, indem mit Boc-D-Ala gemäss dem unten beschriebenen Verfahren begonnen wurde.

Koppeln wird gemäss Schema A wie folgt durchgeführt:

| SCHEMA A | |
|---|---|
| Reagens | Mischzeit (Minuten) |
| 1. Boc-Aminosäure (2-3 m Mol/g Harz) + Aeq. Menge von DIC<br>2. MeOH (zweimal)<br>3. CH$_2$Cl$_2$ (zweimal) | 60 - 90<br>1<br>1 |

Deblockierung wird gemäss Schema B wie folgt durchgeführt:

| SCHEMA B | |
|---|---|
| Reagens | Mischzeit (min.) |
| 4. 50%iges TFA/1%iges Aethanedithiol in CH$_2$Cl$_2$ (zweimal)<br>5. IpOH/1%iges Aethandithiol<br>6. 10%iges TEA in CH$_2$Cl$_2$<br>7. MeOH<br>8. 10%iges TEA in CH$_2$Cl$_2$<br>9. MeOH (zweimal)<br>10. CH$_2$Cl$_2$ (zweimal) | 15 + 15<br>1<br>2<br>1<br>2<br>1 + 1<br>1 + 1 |

Zusammengefasst wird das Boc zum Schutz der alpha-Aminogruppen verwendet. Tos wird verwendet, um die Guanidinogruppe des Arg zu schützen. DCB wird als Schutzgruppe für die phenolische Hydroxylgruppe des Tyr verwendet, und die OH-Gruppe des Ser wird von Bzl geschützt. Ein zwei- bis dreifacher Ueberschuss der geschützten Aminosäure wird während zwei Stunden basierend auf dem NH$_2$-Gehalt des Benzhydrylaminharzes plus eines Aequivalents des DIC in CH$_2$Cl$_2$ oder 10-50%igem DMF/CH$_2$Cl$_2$ je nach Löslichkeit der Boc-Aminosäure verwendet.

Die N-Terminal-Acetylierung wird mit einem 50-fachen Ueberschuss an Essigsäureanhydrid in CH$_2$Cl$_2$ während 0,5 Stunden durchgeführt. Das so erhaltene, geschützte Zwischenpeptid weist folgende Zusammensetzung auf:

Ac-D-Nal(2)-D-Phe(4-Cl)-D-Trp-Ser(X$^4$)-Tyr-(X$^5$)-D-Lys(X$^5$)-Leu-Arg(X$^5$)-Pro-D-Ala-NH-X$^{10}$ worin X$^4$ Bzl ist und X$^5$ DCB, X$^5$ Z(2-Cl), X$^5$ Tos und X$^{10}$ eine im Harz eingebaute Benzhydrylgruppe ist.

Um den geschützten Peptidharz zu spalten und entschützen, wird er mit 1,4 ml m-Cresol und 15 ml Wasserstofffluorid pro Gramm Peptidharz während 0,5 Stunden bei 0°C und 0,5 Stunden bei Raumtemperatur behandelt. Nach der Eliminierung des Wasserstofffluorids unter Hochvakuum wird der Peptidharz mit Diäthyläther gewaschen und das Peptid wird dann mit DMF extrahiert und durch Filtern vom Harz getrennt. Die DMF-Lösung wird dann unter Hochvakuum auf ein kleines Volumen konzentriert und dann mit Diäthyläther trituriert. Das so erhaltene, rohe Produkt wird dann wie oben beschrieben durch vorbereitende HPLC gereinigt. Man erhält so das reine freie Zwischenpeptid der oben angegebenen Formel, worin X$^4$, X$^5$, X$^5$, X$^5$ und X$^{10}$ Wasserstoff bedeuten.

Das freie D-Lys(X$^5$)-enthaltende Zwischenpeptid wird dann bei Raumtemperatur mit Kaliumcyanat in 80 % wässrigem DMF umgesetzt (81 mg KCNO/ml; Zeit: 24 Stunden). Nach Entfernen des Lösungsmittels im Hochvakuum erfolgt Reinigung des Reaktionsproduktes durch präparative HPLC; man erhält so das gewünschte D-Hci-enthaltende Peptid.

Das so erhaltene D-Hci-enthaltende Peptid ist im wesentlichen (zu 95%) rein. Die HPLC-Analyse wird in einem Hewlett-Packard 1090A Gefälle-Flüssig-Chromatographie-System auf einer PHENOMENEX (W-Porex 5C18) Säule durchgeführt und mit Lösungsmitteln eluiert, wobei A: 0,1 %iges TFA, B: 0,1%iges TFA in 70%igem $CH_3CN$ mit einem Gefälle von 30-60% in 30 Minuten. Das gewünschte Peptid weist eine HPLC-Rückhaltezeit (retention time) von 28,2 Minuten auf.

Die Reinigung der Peptide wird auf einem Beckmann Prep-350-Gefälle-Flüssig-Chromatograph durchgeführt, indem eine 41,4 x 250 mm Kartusche mit einer vorbereitenden, umgekehrten DYNEMAX C18-Phase (300A, 12 um) mit Lösungsmitteln verwendet wird, wobei A: 0,1%iges TFA und B: 0,1%iges TFA in 70%igem $CH_3CN$ ist und mit einer Druckgefälle von 45-60% in 30 Minuten. Das reine Peptid, in Form von TFA-Salz erhalten, kann falls erwünscht in die Acetatform umgewandelt werden, und zwar durch Führung durch eine AG3X (Bio-Rad)-Säule in die Acetatform mit nachfolgender Lyophilisierung.

## BEISPIEL II

Ac-D-Nal(2)-D-Phe(4-Cl)-D-Trp-Ser-Tyr-D-Hci(Ethyl)-Leu-Arg-Pro-D-Ala-$NH_2$

Die Herstellung erfolgt aus dem entsprechenden Zwischenpeptid, welches in 6-Stellung anstelle von D-Hci(Ethyl) die Aminosäure D-Lys enthält analog Beispiel I durch Umsetzung mit N-Ethyl-isocyanat (0,1 mg in 10 ml pro 1 g Zwischenpeptid) in DMF bei 0-10° C während 10 Stunden. Die Rückhaltezeit (retention time) ist 30,8 Minuten.

## BEISPIEL III

Die Synthese des Peptids Ac-D-Nal(2)-D-Phe(4-Cl)-D-Trp-Ser-Tyr-D-Cit-Leu-Arg-Pro-D-Ala-$NH_2$ wird wie in Beispiel I beschrieben durchgeführt. Das Ausgangspeptid enthält in 6-Stellung Boc-D-Orn(Z) anstelle von Boc-D-Lys[Z(2-Cl)], welches in Beispiel I verwendet wird. Dieses Ausgangspeptid wird analog Beispiel I hergestellt.
Rückhaltezeit 27,8 Minuten. Rückhaltezeit des Ausgangspeptids 25,5 Minuten.

## BEISPIEL IV

Ac-D-Nal(2)-D-Phe(4-Cl)-D-Trp-Ser-Tyr-D-Cit(Ethyl)-Leu-Arg-Pro-D-Ala-$NH_2$

Dieses Peptid wird aus Ac-D-Nal(2)-Phe(4-Cl)-D-Trp-Ser-Tyr-D-Orn-Leu-Arg-Pro-D-Ala-$NH_2$ und N-Ethyl-isocyanat in DMF (0,1 mg in 10 ml pro Gramm Zwischenpeptid) bei 0-10° C analog Beispiel III erhalten.
Reaktionszeit 10 Stunden.
HPLC-Rückhaltezeit 30,4 Minuten.

## BEISPIEL V

Die Synthese des Peptids Ac-D-Phe(4-Cl)-D-Phe(4-Cl)-D-Trp-Ser-Tyr-D-Hci-Leu-Arg-Pro-D-Ala-$NH_2$ wird wie in Beispiel I beschrieben durchgeführt, mit Ausnahme davon, daß Boc-D-Phe(4-Cl) anstelle von Boc-D-Nal(2) in der Stellung 1 des Ausgangspeptids eingebaut wird, wobei dann als unmittelbares Ausgangspeptid Ac-D-Phe(4-Cl)-D-Phe(4-Cl)-D-Trp-Ser-Tyr-D-Lys-Leu-Arg-Pro-D-Ala-$NH_2$ erhalten wird.
Rückhaltezeit 26,6 Minuten; Rückhaltezeit des Ausgangspeptids mit Ac in 1-Stellung: 24,0 Minuten.

## BEISPIEL VI

Ac-D-Phe(4-Cl)-D-Phe(4-Cl)-D-Trp-Ser-Tyr-D-Hci(Ethyl)-Leu-Arg-Pro-D-Ala-$NH_2$

Ausgangspeptid ist Ac-D-Phe(4-Cl)-Phe(4-Cl)-D-Trp-Ser-Tyr-D-Lys-Leu-Arg-Pro-D-Ala-$NH_2$ (siehe Beispiel V), welches mit N-Ethyl-isocyanat (0,1 mg in 10 ml pro Gramm Ausgangspeptid) bei 0-10° C in DMF umgesetzt wird. Reaktionszeit 10 Stunden.
HPLC-Rückhaltezeit: 29,2 Minuten.

BEISPIEL VII

Die Synthese des Peptids Ac-D-Phe(4-Cl)-D-Phe(4-Cl)-D-Trp-Ser-Tyr-D-Cit-Leu-Arg-Pro-D-Ala-NH$_2$ wird wie in Beispiel I beschrieben durchgeführt mit Ausnahme davon, daß Boc-D-Phe(4-Cl) anstelle des Boc-D-Nal(2) in 1-Stellung und Boc-D-Orn(Z) anstelle von Boc-D-Lys[Z(2-Cl)] in 6-Stellung eingebaut wird, so daß man als unmittelbares Ausgangspeptid Ac-D-Phe(4-Cl)-D-Phe(4-Cl)-D-Trp-Ser-Tyr-D-Orn-Leu-Arg-Pro-D-Ala-NH$_2$ (Rückhaltezeit 24,0 Minuten) erhält, welches dann analog Beispiel I mit KCNO umgesetzt wird.
HPLC-Rückhaltezeit 26,3 Minuten.

BEISPIEL VIII

Ac-D-Phe-(4-Cl)-D-Phe(4-Cl)-D-Trp-Ser-Tyr-D-Cit(Ethyl)-Leu-Arg-Pro-D-Ala-NH$_2$

Ausgangspeptid ist Ac-D-Phe(4-Cl)-D-Phe(4-Cl)-D-Trp-Ser-Tyr-D-Orn-Leu-Arg-Pro-D-Ala-NH$_2$ (siehe Beispiel VII), welches mit N-Ethyl-isocyanat in DMF (0,1 mg in 10 ml pro Gramm Ausgangspeptid) bei 0-10° C während 10 Stunden umgesetzt wird.
HPLC-Rückhaltezeit 28,6 Minuten.

BEISPIEL IX

Die Synthese des Peptids Ac-D-Nal(2)-D-Phe(4-Cl)-D-Trp-Ser-Tyr-D-Hci-Leu-Arg-Pro-Gly-NH$_2$ wird analog Beispiel I durchgeführt. Das erhaltene Peptid weist eine HPLC-Rückhaltezeit von 27,4 Minuten auf.

BEISPIEL X

Ac-D-Nal(2)-D-Phe(4-Cl)-D-Trp-Ser-Tyr-D-Hci(Ethyl)-Leu-Arg-Pro-Gly-NH$_2$

Ausgangspeptid ist: Ac-D-Nal(2)-Phe(4-Cl)-D-Trp-Ser-Tyr-D-Lys-Leu-Arg-Pro-Gly-NH$_2$ (siehe Beispiel IX), welches mit N-Ethyl-isocyanat in DMF (0,1 mg in 10 ml pro Gramm Ausgangspeptid) bei 0-10° C während 10 Stunden umgesetzt wird.
HPLC-Rückhaltezeit 30,0 Minuten.

BEISPIEL XI

Ac-Pro-D-Phe(4-Cl)-D-Trp-Ser-Tyr-D-Hci-Leu-Arg-Pro-D-Ala-NH$_2$

Ausgangspeptid ist: Ac-Pro-D-Phe(4-Cl)-D-Trp-Ser-Tyr-D-Lys-Leu-Arg-Pro-D-Ala-NH$_2$ (Rückhaltezeit 16,8 Minuten), welches analog Beispiel I mit Kaliumcyanat umgesetzt wird. Das Ausgangspeptid wird analog Beispiel I erhalten mit Ausnahme, daß Boc-Pro anstelle von Boc-D-Nal(2) in 1-Stellung eingebaut wird.
HPLC-Rückhaltezeit 19,3 Minuten.

BEISPIEL XII

Ac-Pro-D-Phe(4-Cl)-D-Trp-Ser-Tyr-D-Hci(Ethyl)-Leu-Arg-Pro-D-Ala-NH$_2$

Ausgangspeptid ist:
Ac-D-Pro-Phe(4-Cl)-D-Trp-Ser-Tyr-D-Lys-Leu-Arg-Pro-D-Ala-NH$_2$ (siehe Beispiel XI), welches mit N-Ethyl-isocyanat in DMF (0,1 mg in 10 ml pro Gramm Ausgangspeptid) bei 0-10° C während 10 Stunden umgesetzt wird.
HPLC-Rückhaltezeit 22 Minuten.

BEISPIEL XIII

Ac-Pro-D-Phe(4-Cl)-D-Trp-Ser-Tyr-D-Cit-Leu-Arg-Pro-D-Ala-NH$_2$

Ausgangspeptid ist: Ac-Pro-D-Phe(4-Cl)-D-Trp-Ser-Tyr-D-Orn-Leu-Arg-Pro-D-Ala-NH$_2$ (Rückhaltezeit 16,85 Minuten), welches analog Beispiel I mit Kaliumcyanat umgesetzt wird. Das Ausgangspeptid wird ebenfalls analog Beispiel I erhalten mit dem unterschied, daß Boc-Pro anstelle von Boc-D-Nal(2) in die 1-Stellung und Boc-D-Orn(Z) anstelle von Boc-D-Lys[Z(2-Cl)] in die 6-Stellung eingebaut werden. HPLC-Rückhaltezeit 18,8 Minuten.

BEISPIEL XIV

Ac-Pro-D-Phe(4-Cl)-D-Trp-Ser-Tyr-D-Cit(Ethyl)-Leu-Arg-Pro-D-Ala-NH$_2$

Ausgangspeptid ist: Ac-Pro-D-Phe(4-Cl)-D-Trp-Ser-Tyr-D-Orn-Leu-Arg-Pro-D-Ala-NH$_2$ (siehe Beispiel XIII), welches analog Beispiel VI mit N-Ethyl-isocyanat umgesetzt wird. HPLC-Rückhaltezeit 24,9 Minuten.

BEISPIEL XV

Ac-D-Phe-D-Phe(4-Cl)-D-Trp-Ser-Tyr-D-Hci-Leu-Arg-Pro-D-Ala-NH$_2$

Ausgangspeptid ist: Ac-D-Phe-Phe(4-Cl)-D-Trp-Ser-Tyr-D-Lys-Leu-Arg-Pro-D-Ala-NH$_2$ (Rückhaltezeit 20,8 Minuten), welches analog Beispiel I mit Kaliumcyanat umgesetzt wird. Das Ausgangspeptid wird analog Beispiel I erhalten, wobei jedoch in 1-Stellung anstelle von Boc-D-Nal(2) der Aminosäurerest Boc-D-Phe eingebaut wird. HPLC-Rückhaltezeit 23,4 Minuten.

BEISPIEL XVI

Ac-D-Phe-D-Phe(4-Cl)-D-Trp-Ser-Tyr-D-Hci(Ethyl)-Leu-Arg-Pro-D-Ala-NH$_2$

Ausgangspeptid ist: Ac-D-Phe-D-Phe(4-Cl)-D-Trp-Ser-Tyr-D-Lys-Leu-Arg-Pro-D-Ala-NH$_2$ (siehe Beispiel XV), welches mit N-Ethyl-isocyanat in DMF (0.1 mg in 10 ml pro Gramm Ausgangspeptid) bei 0-10° C während 10 Stunden umgesetzt wird. HPLC-Rückhaltezeit 26,0 Minuten.

BEISPIEL XVII

Ac-D-Phe-D-Phe(4-Cl)-D-Trp-Ser-Tyr-D-Cit-Leu-Arg-Pro-D-Ala-NH$_2$

Ausgangspeptid ist: Ac-D-Phe-D-Phe(4-Cl)-D-Trp-Ser-Tyr-D-Orn-Leu-Arg-Pro-D-Ala-NH$_2$ (Rückhaltezeit 21,0 Minuten), welches analog Beispiel I mit Kaliumcyanat umgesetzt wird. Herstellung des Ausgangspeptids erfolgt analog Beispiel I mit dem Unterschied, daß anstelle von Boc-D-Nal(2) in 1-Stellung Boc-D-Phe und anstelle von Boc-D-Lys[Z(2-Cl)] in 6-Stellung Boc-D-Orn(Z) eingebaut werden. HPLC-Rückhaltezeit 23,1 Minuten.

BEISPIEL XVIII

Ac-D-Phe-D-Phe(4-Cl)-D-Trp-Ser-Tyr-D-Cit(Ethyl)-Leu-Arg-Pro-D-Ala-NH$_2$

Ausgangspeptid ist: Ac-D-Phe-D-Phe(4-Cl)-D-Trp-Ser-Tyr-D-Orn-Leu-Arg-Pro-D-Ala-NH$_2$ (siehe Beispiel XVII), welches mit N-Ethyl-isocyanat in DMF (0,1 mg in 10 ml pro Gramm Ausgangspeptid) bei 0-10° C während 10 Stunden umgesetzt wird. HPLC-Rückhaltezeit 25,4 Minuten.

BEISPIEL XIX

Beispiel für die allgemeine Herstellung eines erfindungsgemäßen Peptids der Formel Ac-D-Nal(2)-D-Phe(4-Cl)-D-Typ-Ser-Tyr-D-Hci-Leu-Arg-Pro-D-Ala-NH$_2$.

Dieses Peptid wird Schritt für Schritt mittels eines Benzhydrylaminharzes, welches ungefähr 1,0 mol äquivalente Aminogruppen pro Gramm enthält unter Verwendung eines Beckman 990 Synthesizers, wobei mit Boc-D-Ala begonnen wird. Die Kupplung erfolgt gemäß Schema A, welches in Beispiel I angegeben ist. Die D-Blockierung wird ausgeführt gemäß dem Schema B, welches in Beispiel I angegeben ist. Für die übrigen Bedingungen gelten die entsprechenden Angaben gemäß Beispiel I.

Das so erhaltene geschützte Zwischenpeptid hat die folgende Zusammensetzung:
Ac-D-Nal(2)-D-Phe(4-Cl)-D-Trp-Ser(X$^4$)-Tyr(X$^5$)-D-Hci-Leu-Arg(X$^5$)-Pro-D-Ala-NHX$^{10}$,wobei X$^4$ = Bzl und X$^5$ = Dzb, X$^5$ = Tos und X$^{10}$ eine Benzhydrylgruppe, die in dem Harz eingebaut ist, darstellt.

Die Spaltung des so erhaltenen geschützten Peptids erfolgt gemäß Beispiel I mit 1,4 ml m-Cresol und 15 ml Fluorwasserstoff pro Gramm Peptidharz (0,5 Stunden bei 0° C und 0,5 Stunden bei Raumtemperatur). Alle übrigen Maßnahmen wie in Beispiel I angegeben. Das erhaltene Rohprodukt wird durch preparative HPLC-Chromatographie gereinigt. Die HPLC-Analyse wird entsprechend Beispiel I in einem Hewlett-Packard 1090A, Gefälle-Flüssig-Chromatographie-System unter Verwendung einer Phenomenex-Säule ausgeführt mit folgenden Losungsmitteln eluiert:

Lösungsmittel A: 0,1% TFA, Lösungsmittel B: 0,1% TFA in 70 % CH$_3$CN mit einem Gradienten von 35 - 75 % in 30 Minuten. Das erhaltene Dipeptid, welches in der 6-Stellung D-Hci enthält, hat eine HPLC-Rückhaltezeit von 22,9 Minuten. Weitere Reinigung erfolgt auf einen Beckmann-Prep-350-Gefälle-Flüssig-Chromatograph entsprechend Beispiel I.

BEISPIEL XX

Das Peptid H$_2$N-CO-D-Nal(2)-D-Phe(4-Cl)-D-Trp-Ser-Tyr-D-Hci-Leu-Arg-Pro-D-Ala-NH$_2$ wird gemäß Beispiel XIX erhalten mit dem Unterschied, daß anstelle von Boc-D-Nal(2) in die 1-Stellung H$_2$N-CO-D-Nal(2) eingebaut wird und die N-terminale Acetylierung nicht erfolgt. HPLC-Rückhaltezeit 24,0 Minuten.

BEISPIEL XXI

Die Synthese des Peptids Ac-D-Nal(2)-D-Phe(4-Cl)-D-Trp-Ser-Tyr-D-Cit-Leu-Arg-Pro-D-Ala-NH$_2$ wird wie in Beispiel XIX beschrieben durchgeführt mit Ausnahme davon, daß Boc-D-Cit anstelle von Boc-D-Hci in Stellung 6 eingebaut wird. Das gewünschte Peptid weist eine HPLC-Rückhaltezeit von 22,5 Minuten auf.

BEISPIEL XXII

Ac-D-Phe(4-Cl)-D-Phe(4-Cl)-D-Trp-Ser-Tyr-D-Hci-Leu-Arg-Pro-D-Ala-NH$_2$

Herstellung gemäß Beispiel XIX mit dem Unterschied, daß BOC-D-Phe(4-Cl) anstelle von Boc-D-Nal(2) in 1-Stellung eingebaut wird.
HPCL-Rückhaltezeit 24.0 Minuten.

BEISPIEL XXIII

Ac-D-Phe(4-Cl)-D-Phe(4-Cl)-D-Trp-Ser-Tyr-D-Cit-Leu-Arg-Pro-D-Ala-NH$_2$

Die Herstellung erfolgt analog Beispiel XIX mit dem Unterschied, daß Boc-D-Phe(4-Cl) anstelle von Boc-D-Nal(2) in die 1-Stellung und Boc-D-Cit anstelle von Boc-D-Hci in 6-Stellung eingebaut wird.
Rückhaltezeit des erhaltenen Peptids 20,8 Minuten.

BEISPIEL XXIV

Ac-D-Nal(2)-D-Phe(4-Cl)-D-Trp-Ser-Tyr-D-Hci-Leu-Arg-Pro-Gly-NH$_2$

Die Herstellung erfolgt entsprechend Beispiel XIX mit dem Unterschied, daß Boc-Gly anstelle von Boc-D-Ala in die 10-Stellung eingebaut wird.
Das erhaltene Peptid hat eine HPLC-Rückhaltezeit von 22,4 Minuten.

BEISPIEL XXV

Ac-D-Nal(2)-D-Phe(4-Cl)-D-Pal(3)-Ser-Tyr-D-Hci-Leu-Arg-Pro-D-Ala-NH$_2$

Herstellung erfolgt gemäß Beispiel XIX mit dem Unterschied, daß Boc-D-Pal(3) anstelle von Boc-D-Trp in die 3-Stellung eingebaut wird.
Das erhaltene Peptid hat eine HPLC-Rückhaltezeit von 13,6 Minuten.

BEISPIEL XXVI

Ac-D-Nal-D-Phe(4-Cl)-D-Pal(3)-Ser-Tyr-D-Cit-Leu-Arg-Pro-D-Ala-NH$_2$

Herstellung erfolgt entsprechend Beispiel XIX mit dem Unterschied, daß Boc-D-Cit anstelle von D-Hci in die 6-Stellung und Boc-D-Pal(3) anstelle von Boc-D-Trp in die 3-Stellung eingebaut wird.
Das erhaltene Peptid hat eine HPLC-Rückhaltezeit von 13,3 Minuten.

BEISPIEL XXVII

Die Synthese des Peptids H$_2$N-CO-D-Nal(2)-D-Phe(4-Cl)-D-Pal(3)-Ser-Tyr-D-Cit-Leu-Arg-Pro-D-Ala-NH$_2$ wird wie in Beispiel XIX beschrieben durchgeführt mit dem Unterschied, daß Boc-D-Cit anstelle von Boc-D-Hci in Stellung 6 eingebaut wird, daß Boc-D-Pal(3) anstelle von Boc-D-Trp in Stellung 3 eingebaut wird und daß die N-terminale-Acetylierung ausgelassen wird.
Man erhält so das Zwischenpeptid:
H-D-Nal(2)-D-Phe(4-Cl)-D-Pal(3)-Ser-Tyr-D-Cit-Leu-Arg-Pro-D-Ala-NH$_2$. Das so erhaltene, freie Peptid wird dann mit Kaliumcyanat in 80%igem wässrigen DMF (81 mg KOCN/300 mg Peptid/ml) bei Raumtemperatur während 24 Stunden umgesetzt. Nach Verdampfung unter Hochvakuum wird die Reaktionsmischung durch vorbereitende HPLC gereinigt. Das so erhaltene Peptid hat eine HPLC-Rückhaltezeit von 14.4 Minuten.

BEISPIEL XXVIII

H$_2$N-CO-D-Nal(2)-D-Phe(4-Cl)-D-Pal(3)-Ser-Tyr-D-Hci-Leu-Arg-Pro-D-Ala-NH$_2$ wird analog Beispiel XIX durchgeführt mit dem Unterschied, daß Boc-D-Pal(3) anstelle von Boc-D-Trp in Stellung 3 eingebaut wird, und daß die N-terminale-Acetylierung ausgelassen wird. Man erhält das Zwischenpeptid H-D-Nal(2)-D-Phe-(4-Cl)-D-Pal(3)-Ser-Tyr-D-Hci-Leu-Arg-Pro-D-Ala-NH$_2$. Das so erhaltene freie Peptid wird dann mit Kaliumcyanat in wässrigem DMF (81 mg KOCN/300 mg Peptid/ml) bei Raumtemperatur während 24 Stunden umgesetzt. Nach Verdampfung unter Hochvakuum wird die Reaktionsmischung durch präparative HPLC gereinigt. Das so erhaltene Peptid hat eine HPLC-Rückhaltezeit von 14,7 Minuten.

BEISPIEL XXIX

Die Synthese des Peptids H$_2$N-CO-D-Nal(2)-D-Phe(4-Cl)-D-Trp-Ser-Tyr-D-Cit-Leu-Arg-Pro-D-Ala-NH$_2$ beginnt mit der Herstellung des Zwischenpeptids H-D-Nal(2)-D-Phe-(4-Cl)-D-Trp-Ser-Tyr-D-Cit-Leu-Arg-Pro-D-Ala-NH$_2$. Die Synthese des Zwischenpeptids wird wie in Beispiel XIX beschrieben ist durchgeführt mit dem Unterschied, daß Boc-D-Orn(Z) in Stellung 6 anstelle von Boc-D-Hci eingebaut wird, und daß die N-terminale-Acetylierung ausgelassen wird. Das freie in 6-Stellung D-Orn enthaltende Peptid wird dann mit Kaliumcyanat in 80%igem wässrigen DMF (162 mg KOCN/300 mg Peptid/ml) bei Raumtemperatur während 24 Stunden umgesetzt. Nach Verdampfung unter Hochvakuum wird die Reaktionsmischung durch präparative HPLC gereinigt. Das so erhaltene Peptid hat eine HPLC-Rückhaltezeit von 23,6 Minuten.

BEISPIEL XXX

Die Synthese des Peptids H$_2$N-CO-D-Nal(2)-D-Phe(4-Cl)-D-Trp-Ser-Tyr-D-Hci-Leu-Arg-Pro-D-Ala-NH$_2$ beginnt mit der Herstellung des Zwischenpeptids H-D-Nal(2)-D-Phe-(4-Cl)-D-Trp-Ser-Tyr-D-Lys-Leu-Arg-Pro-D-Ala-NH$_2$. Die Synthese des Zwischenpeptids wird wie in Beispiel XIX beschrieben ist durchgeführt mit dem Unterschied, daß Boc-D-Lys[Z(2-Cl)] anstelle von Boc-D-Hci in Stellung 6 eingebaut wird, und daß die N-terminale-Acetylierung ausgelassen wird. Das freie Peptid, welches in 6-Stellung D-Lys enthält, wird dann mit Kaliumcyanat in 80%igem wässrigen DMF (164 mg KOCN/300 mg Peptid/ml) bei Raumtemperatur während 24 Stunden zum Reagieren gebracht. Nach Verdampfung unter Hochvakuum wird die Reaktionsmischung durch präparative HPLC gereinigt. Das so erhaltene Peptid hat eine HPLC-Rückhaltezeit von 14,0 Minuten.

BEISPIEL XXXI

Die Tablettenformulierung für die bukkale (z.B. sublinguale) Verabreichung:
1. LHRH-Antagonist 10,0 mg
pressbarer Zucker 86,0 mg

Kalciumstearat 4,0 mg
2. LHRH-Antagonist 10,0 mg
pressbarer Zucker 88,5 mg
Magnesiumstearat 1,5 mg
3. LHRH-Antagonist 5,0 mg
Mannitt 83,5 mg
Magnesiumstärke 1,5 mg
4. LHRH-Antagonist 10,0 mg
Vorgelatinierte Stärke 10,0 mg
Lactose 74,5 mg
Vorgelatinierte Stärke 15,0 mg
Magnesiumstearat, USP 1,5 mg

Verfahren A. LHRH-Antagonist wird in einer ausreichenden Menge Wasser gelöst, um ein nasses Granulat zu bilden, wenn er mit dem Zuckeranteil des Trägers gemischt wird. Nach vollständigem Mischen wird das Granulat in einer Tropfschale einer Flüssigbett-Trockenvorrichtung getrocknet. Das trockene Granulat wird dann durchleuchtet, um eventuelle grossen Aggregate aufzuzeigen und diese zu zerkleinern und dann mit den übrigen Komponenten gemischt. Das Granulat wird dann in einer Standard-Tablettenmaschine auf ein spezifisches Tablettengewicht zusammengepresst.

Verfahren B. Bei dieser Herstellungsmethode enthalten alle Formulierungen 0,01%ige Gelatine. Die Gelatine wird zuerst im wässrigen Granulatlösungsmittel gelöst, dann wird der LHRH-Antagonist zugegeben. Die übrigen Stufen sind wie bei Methode A beschrieben.

BEISPIEL XXXII

Langwirkende, intramuskulär injektzierbare Formulierung

Langwirkendes intramuskulär-injektzierbares Semsamöl-Gel
LHRH-Antagonist 10,0 mg
Aluminiummonostearat 20,0 mg
Sesamöl ad 10 ml.

Das Aluminiummonostearat wird zusammen mit dem Sesamöl lange unter Rühren auf 125°C erhitzt, bis eine klare, gelbe Lösung erhalten wird. Diese Mischung wird dann im Sterilisationsapparat sterilisiert und abgekühlt. Der LHRH-Antagonist wird dann aseptisch mit Hilfe von Triturierung beigefügt. Besonders bevorzugte LHRH-Antagonisten sind Salze mit niedriger Löslichkeit, z.B. Zinksalze, Zinktannatsalze, Pamoatsalze und ähnliche. Diese weisen eine aussergewöhnlich lange Aktivitätsdauer auf.

BEISPIEL XXXIII

Langwirkender intramuskulär-injektzierbare - bioabbaubare Polymer-Mikrokapseln

LHRH-Antagonisten 1%
25/75 Glycolid/Lactid Copolymer (Copolymerisat aus Glycolsäure und Milchsäure) (0,5 intrinsische Viskosität) 99 %
Mikrokapseln (0-150 Angström) der oben genannten Formulierung suspendiert in:
Dextrose 5,0%
Carboxymethylcellulose-Natrium 0,5 %
Benzylalkohol 0,9%
Tween 80 0,1%
Wasser, gereinigt q.w. (soviel wie nötig für) 100,0% 25 mg Mikrokapseln werden in 1,0 ml des Vehikels suspendiert.

BEISPIEL XXXIV

Wässrige Lösung einer intramuskulären Injektion

LHRH-Antagonist 500 mg
Gelatine(nichtantigenisch)5 mg
Wasser für die Injektionszwecke ad 100 ml

22

Die Gelatine und der LHRH-Antagonist werden im Injektionswasser aufgelöst. Danach wird die Lösung steril gefiltert.

BEISPIEL XXXV

Formulierung zur rektalen Verabreichung
Suppositorvehikel zur rektalen Verabreichung
LHRH-Antagonist 5,0 mg
Witepsol H15 20,0 mg

Der LHRH-Antagonist wird mit dem geschmolzenen Witepsol H15 gemischt und in eine Gussform von 2 Gramm gegossen.

**Patentansprüche**

1.  Peptid der Formel:

    $X-R^1-R^2-R^3-Ser-Tyr-R^6-Leu-Arg-Pro-R^{10}-NH_2$      I

    worin

    X eine Acylgruppe aus geraden oder verzweigten Ketten aliphatischer oder alicyklischer Carbonsäuren mit 1 bis 7 Hohlenstoffatomen oder eine Carbamoyl-Gruppe ist,
    $R^1$ D- oder L-Pro, D- oder $L-\Delta^3$-Pro, D-Phe, D-Phe(4-Hl), D-Ser, D-Thr, D-Ala, D-Nal(1) oder D-Nal(2) ist,
    $R^2$ D-Phe oder D-Phe(4-Hl) ist,
    $R^3$ D-Trp, D-Phe, D-Pal(3), D-Nal(1) oder D-Nal(2) ist,
    $R^6$ D-Cit, D-Hci, D-Cit(Q) oder D-Hci(Q) ist und
    $R^{10}$ Gly oder D-Ala ist,
    wobei Hl Fluor, Chlor oder Brom und Q $C_1-C_3$-Alkyl ist,
    und pharmazeutisch annehmbaren Säureadditionssalze davon.

2.  Peptide nach Anspruch 1, dadurch gekennzeichnet, dass
    X eine Acetyl- oder Carbamoyl-Gruppe ist,
    $R^1$ D-Nal(2), D-oder L-Pro, D-Phe oder D-Phe(4-Cl) ist,
    $R^2$ D-Phe(4-Cl) oder D-PHe(4-F) und
    $R^3$ D-Trp oder D-Pal(3)ist.

3.  Peptide der Formel:

    $X_1-R^1-R^2-R^3-Ser(X^4)-Tyr(X^5)-R^{*6}-Leu-Arg(X^5)-Pro-R^{10}-NH-X^{10}$

    worin
    $X_1$ eine Acylgruppe aus geraden oder verzweigten Ketten aliphatischer oder alicyklischer Carbonsäuren mit 1 bis 7 Kohlenstoffatomen oder t-Boc, Wasserstoff oder Carbamoyl ist, $X^4$ Wasserstoff oder eine Schutzgruppe für die Ser-Hydroxylgruppe ist,
    $X^5$ Wasserstoff oder eine Schutzgruppe für die Tyr-Phenolhydroxylgruppe ist,
    $X^5$ Wasserstoff oder eine Schutzgruppe für die Arg-Guanidinogruppe ist,
    $X^{10}$ Wasserstoff oder ein Trägerharz mit Benzhydryl- oder Methylbenzhydrylgruppen ist,
    $R^1$ D- oder L-Pro, D- oder $L-\Delta^3$-Pro, D-Phe, D-Phe(4-Hl), D-Ser, D-Thr, D-Ala, D-Nal(1) oder D-Nal(2) ist,
    $R^2$ D-Phe oder D-Phe(4-Hl) ist,
    $R^3$ D-Trp, D-Phe, D-Pal(3), D-Nal(1) oder D-Nal(2) ist,
    $R^{*6}$ D-Cit oder D-Hci ist und
    $R^{10}$ Gly oder D-Ala ist,
    wobei Hl Fluor, Chlor oder Brom ist.

4.  Peptide nach einem der Ansprüche 1 bis 3, dadurch gekennzeichnet, daß
    X Acetyl, Boc oder Wasserstoff ist,
    $X^4$ Benzyl ist,
    $X^5$ 2,6-Dichlorbenzyl ist,
    $X^5$ Benzyloxycarbonyl oder 2-Chlorbenzyloxycarbonyl ist,

$X^8$ p-Toluolsulphonyl oder Nitro ist,

$X^{10}$ ein Benzhydryl oder Methylbenzhydrylgruppen enthaltendes Trägerharz ist,

$R^1$ D-Nal(2) oder D-Phe(4-Cl) ist,

$R^2$ D-Phe(4-Cl) ist,

$R^3$ D-Trp oder D-Pal(3) ist,

$R^{10}$ D-Ala ist.

**5.** Peptid nach einem oder mehreren der vorangegangenen Ansprüche, dadurch gekennzeichnet, daß X oder $X_1$ Acetyl ist.

**6.** Peptid nach einem oder mehreren der vorangegangenen Ansprüche, dadurch gekennzeichnet, daß X oder $X_1$ Carbamoyl ist.

**7.** Peptid nach einem oder mehreren der vorangegangenen Ansprüche, dadurch gekennzeichnet, daß $R^6$ oder $R^{*6}$ D-Cit oder D-Hci ist.

**8.** Verfahren zur Herstellung von Peptiden der Formel:

$$X-R^1-R^2-R^3-Ser-Tyr-R^6-Leu-Arg-Pro-R^{10}-NH_2 \qquad I$$

worin

X eine Acylgruppe aus geraden oder verzweigten Ketten aliphatischer oder alicyclischer Carbonsäuren mit 1 bis 7 Kohlenstoffatomen oder eine Carbamoyl-Gruppe ist.

$R^1$ D- oder L-Pro, D-oder L-$\Delta^3$-Pro, D-Phe, D-Phe(4-Hl), D-Ser, D-Thr, D-Ala, D-Nal(1) oder D-Nal(2) ist,

$R^2$ D-Phe oder D-Phe(4-Hl) ist,

$R^3$ D-Trp, D-Phe, D-Pal(3), D-Nal(1) oder D-Nal(2) ist,

$R^6$ D-Cit, D-Hci, D-Cit(Q) oder D-Hci(Q) ist und

$R^{10}$ Gly oder D-Ala ist,

wobei Hl Fluor, Chlor oder Brom und Q $C_1$-$C_3$-Alkyl ist, und deren pharmazeutisch annehmbaren Säureadditionssalzen,

dadurch gekennzeichnet, daß man sie durch in der Peptidchemie übliche Fragmentkondensation von entsprechenden Peptidbruchstücken oder durch in der Peptidchemie üblichen stufenweisen Aufbau herstellt oder daß man in einem Peptid der Formel I, worin $R^6$ D-Orn oder D-Lys ist, die freie Aminogruppe von D-Orn oder D-Lys durch Umsetzung mit einem Alkalicyanat oder einer Cyanat-liefernden Verbindung oder einem $C_1$-$C_3$-Alkylisocyanat zur Ureidogruppe umsetzt und gegebenenfalls die erhaltenen Peptide acyliert und/oder in die Amide überführt und gegebenenfalls in physiologisch unbedenkliche Säuresalze überführt.

**9.** Verfahren nach Anspruch 9,

dadurch gekennzeichnet, daß man den stufenweisen Aufbau durchführt, indem man zunächst die Carboxyterminale Aminosäure Glycin oder D-Alanin beziehungsweise deren Amide, deren $\alpha$-ständige Aminogruppe geschützt ist, an einen hierfür üblichen synthetischen Träger kovalent bindet, die $\alpha$-Amino-Schutzgruppe abspaltet, an die so erhaltene freie Aminogruppe die nächstfolgende geschützte Aminosäure über ihre Carboxy-Gruppe bindet, dann wiederum die $\alpha$-Amino-Schutzgruppe dieser zweiten Aminosäure abspaltet, hieran die nächste Aminosäure bindet und in dieser Weise Schritt für Schritt die übrigen Aminosäuren des zu synthetisierenden Peptids in der richtigen Reihenfolge verknüpft und nach Verknüpfung aller Aminosäuren das fertige Peptid gemäß Anspruch 1 vom Träger abspaltet und gegebenenfalls weitere vorhandene Seitenfunktions-Schutzgruppen abspaltet.

**10.** Verbindungen gemäß Anspruch 1 oder 2 zur Anwendung als therapeutische Wirkstoffe.

**11.** Arzneimittel, enthaltend eine Verbindung der allgemeinen Formal I gemäß Anspruch 1 oder 2 neben üblichen Träger- und/oder Verdünnungsbeziehungsweise Hilfsstoffen.

**12.** Verfahren zur Herstellung eines Arzneimittels, dadurch gekennzeichnet, daß eine Verbindung I gemäß Anspruch 1 oder 2 mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

13. Verfahren nach Anspruch 12 oder 13, dadurch gekennzeichnet, daß die Menge an Wirkstoff gemäß einem oder mehreren der vorangegangenen Ansprüche 1 bis 8 zwischen 0,1 und 2,5 mg/kg Körpergewicht pro Tag liegt.

14. Verwendung von Verbindungen gemäß Anspruch 1 oder 2 zur Herstellung von Arzneimitteln.

**Claims**

1. A peptide of the formula

   X-$R^1$-$R^2$-$R^3$-Ser-Tyr-$R^6$-Leu-Arg-Pro-$R^{10}$-$NH_2$     I

   wherein
   X is an acyl group consisting of linear or branched chains of aliphatic or alicyclic carboxylic acids having 1 to 7 carbon atoms, or a carbamoyl group,
   $R^1$ is D- or L-Pro, D- or L-$\Delta^3$-Pro, D-Phe, D-Phe(4-Hl),
   D-Ser, D-Thr, D-Ala, D-Nal(1) or D-Nal(2),
   $R^2$ is D-Phe or D-Phe(4-Hl),
   $R^3$ is D-Trp, D-Phe, D-Pal(3), D-Nal(1) or D-Nal(2),
   $R^6$ is D-Cit, D-Hci, D-Cit(Q) or D-Hci(Q) and
   $R^{10}$ is Gly or D-Ala,
   Hl being fluorine, chlorine or bromine and Q being $C_1$- $C_3$-alkyl,
   and pharmaceutically acceptable acid addition salts thereof.

2. Peptides according to Claim 1, characterized in that
   X is an acetyl or carbamoyl group,
   $R^1$ is D-Nal(2), D- or L-Pro, D-Phe or D-Phe(4-Cl),
   $R^2$ is D-Phe(4-Cl) or D-Phe(4-F) and
   $R^3$ is D-Trp or D-Pal(3).

3. Peptides of the formula

   $X_1$-$R^1$-$R^2$-$R^3$-Ser($X^4$)-Tyr($X^5$)-$R^{*6}$-Leu-Arg($X^5$)-Pro-$R^{10}$-NH-$X^{10}$

   wherein
   $X_1$ is an acyl group consisting of linear or branched chains of aliphatic or alicyclic carboxylic acids having 1 to 7 carbon atoms, or t-Boc, hydrogen or carbamoyl,
   $X^4$ is hydrogen or a protecting group for the Ser hydroxyl group,
   $X^5$ is hydrogen or a protecting group for the Tyr phenolic hydroxyl group,
   $X^5$ is hydrogen or a protecting group for the Arg guanidino group,
   $X^{10}$ is hydrogen or a carrier resin having benzhydryl or methylbenzhydryl groups,
   $R^1$ is D- or L-Pro, D- or L-$\Delta^3$-Pro, D-Phe, D-Phe(4-Hl),
   D-Ser, D-Thr, D-Ala, D-Nal(1) or D-Nal(2),
   $R^2$ is D-Phe or D-Phe(4-Hl),
   $R^3$ is D-Trp, D-Phe, D-Pal(3), D-Nal(1) or D-Nal(2),
   $R^{*6}$ is D-Cit or D-Hci and
   $R^{10}$ is Gly or D-Ala,
   Hl being fluorine, chlorine or bromine.

4. Peptides according to one of Claims 1 to 3, characterized in that
   X is acetyl, Boc or hydrogen,
   $X^4$ is benzyl,
   $X^5$ is 2,6-dichlorobenzyl,
   $X^5$ is benzyloxycarbonyl or 2-chlorobenzyloxycarbonyl,
   $X^5$ is p-toluenesulphonyl or nitro,
   $X^{10}$ is a carrier resin containing benzhydryl or methylbenzhydryl groups,
   $R^1$ is D-Nal(2) or D-Phe(4-Cl),
   $R^2$ is D-Phe(4-Cl),

$R^3$ is D-Trp or D-Pal(3) and
$R^{10}$ is D-Ala.

5. A peptide according to one or more of the preceding claims, characterized in that X or $X_1$ is acetyl.

6. A peptide according to one or more of the preceding claims, characterized in that X or $X_1$ is carbamoyl.

7. A peptide according to one or more of the preceding claims, characterized in that $R^6$ or $R^{*6}$ is D-Cit or D-Hci.

8. A method of preparing peptides of the formula

$$X-R^1-R^2-R^3-Ser-Tyr-R^6-Leu-Arg-Pro-R^{10}-NH_2 \qquad I$$

wherein
X is an acyl group consisting of linear or branched chains of aliphatic or alicyclic carboxylic acids having 1 to 7 carbon atoms, or a carbamoyl group,
$R^1$ is D- or L-Pro, D- or L-$\Delta^3$-Pro, D-Phe, D-Phe(4-Hl),
D-Ser, D-Thr, D-Ala, D-Nal(1) or D-Nal(2),
$R^2$ is D-Phe or D-Phe(4-Hl),
$R^3$ is D-Trp, D-Phe, D-Pal(3), D-Nal(1) or D-Nal(2),
$R^6$ is D-Cit, D-Hci, D-Cit(Q) or D-Hci(Q) and
$R^{10}$ is Gly or D-Ala,
Hl being fluorine, chlorine or bromine and Q being $C_1$-$C_3$-alkyl,
and pharmaceutically acceptable acid addition salts thereof,
characterized in that they are prepared by fragment condensation of corresponding peptide fragments, as conventionally used in peptide chemistry, or by stepwise synthesis as conventionally used in peptide chemistry, or in that, in a peptide of formula I wherein $R^6$ is D-Orn or D-Lys, the free amino group of D-Orn or D-Lys is converted to the ureido group by reaction with an alkali metal cyanate or a cyanate- providing compound or a $C_1$-$C_3$-alkyl isocyanate, and, optionally, the peptides obtained are acylated and/or converted to the amides and, optionally, converted to physiologically acceptable acid salts.

9. A method according to Claim 8, characterized in that the stepwise synthesis is carried out by a procedure in which firstly the carboxy-terminal amino acid glycine or D-alanine or the amides thereof, whose amino group in the $\alpha$-position is protected, is covalently bonded to a synthetic carrier conventionally used for this purpose, the $\alpha$-amino protecting group is cleaved, the next protected amino acid is bonded via its carboxyl group to the free amino group obtained in this way, the $\alpha$-amino protecting group of this second amino acid is then cleaved in turn, the next amino acid is bonded thereto and the remaining amino acids of the peptide to be synthesized are linked stepwise in this way in the correct sequence and, after all the amino acids have been linked, the finished peptide according to Claim 1 is cleaved from the carrier and, optionally, other side-group protecting groups present are cleaved.

10. Compounds according to Claim 1 or Claim 2 for use as therapeutic active ingredients.

11. A drug containing a compound of general formula I according to Claim 1 or Claim 2, together with conventional excipients and/or diluents or adjuncts.

12. A method of preparing a drug, characterized in that a compound I according to Claim 1 or Claim 2 is processed to pharmaceutical formulations with customary pharmaceutical excipients and/or diluents or other adjuncts, or is converted to a form usable in therapy.

13. A method according to Claim 11 or Claim 12, characterized in that the amount of active ingredient according to one or more of the preceding Claims 1 to 8 is between 0.1 and 2.5 mg/kg of body weight per day.

14. Use of compounds according to Claim 1 or Claim 2 for the preparation of drugs.

**Revendications**

1. Peptide de formule

X-R$^1$-R$^2$-R$^3$-Ser-Tyr-R$^6$-Leu-Arg-Pro-R$^{10}$-NH$_2$     I

dans laquelle

X        est un groupement acyle composé de chaînes droites ou ramifiées d'acides carboxyliques aliphatiques ou alicycliques à 1-7 atomes de carbone, ou un groupement carbamoyle,

R$^1$        est mis pour D- ou L-Pro, D- ou L-$\Delta^3$-Pro, D-Phe, D-Phe(4-Hl), D-Ser, D-Thr, D-Ala, D-Nal(1) ou D-Nal(2),

R$^2$        est mis pour D-Phe ou D-Phe(4-Hl),

R$^3$        est mis pour D-Trp, D-Phe, D-Pal(3), D-Nal(1) ou D-Nal(2),

R$^6$        est mis pour D-Cit, D-Hci, D-Cit(Q) ou D-Hci(Q) et

R$^{10}$        est mis pour Gly ou D-Ala,

Hl        désignant un atome de fluor, de chlore ou de brome et

Q        désignant un reste alkyle en C$_1$-C$_3$,

et sels d'acides d'addition de ce peptide acceptables pharmaceutiquement.

2. Peptides selon la revendication 1, caractérisés en ce que

X        est un groupement acétyle ou carbamoyle,

R$^1$        est mis pour D-Nal(2), D- ou L-Pro, D-Phe ou D-Phe(4-Cl),

R$^2$        est mis pour D-Phe(4-Cl) ou D-Phe(4-F) et

R$^3$        est mis pour D-Trp ou D-Pal(3).

3. Peptides de formule

X$_1$-R$^1$-R$^2$-R$^3$-Ser(X$^4$)-Tyr(X$^5$)-R$^{*6}$-Leu-Arg(X$^5$)-Pro-R$^{10}$-NH-X$^{10}$

dans laquelle

X$_1$        est un groupement acyle composé de chaînes droites ou ramifiées d'acides carboxyliques aliphatiques ou alicycliques à 1-7 atomes de carbone, t-Boc, un atome d'hydrogène ou un groupement carbamoyle,

X$^4$        est un atome d'hydrogène ou un groupement protecteur pour le groupement hydroxyle de Ser,

X$^5$        est un atome d'hydrogène ou un groupement protecteur pour le groupement phénolhydroxyle de Tyr,

X$^5$        est un atome d'hydrogène ou un groupement protecteur pour le groupement guanidyle d'Arg,

X$^{10}$        est un atome d'hydrogène ou une résine porteuse à groupements benzhydryle ou méthylbenzhydryle,

R$^1$        est mis pour D- ou L-Pro, D- ou L-$\Delta^3$-Pro, D-Phe, D-Phe(4-Hl), D-Ser, D-Thr, D-Ala, D-Nal(1) ou D-Nal(2),

R$^2$        est mis pour D-Phe ou D-Phe(4-Hl),

R$^3$        est mis pour D-Trp, D-Phe, D-Pal(3), D-Nal(1) ou D-Nal(2),

R$^{*6}$        est mis pour D-Cit ou D-Hci et

R$^{10}$        est mis pour Gly ou D-Ala,

Hl        désignant un atome de fluor, de chlore ou de brome,

4. Peptides selon l'une quelconque des revendications 1 à 3, caractérisés en ce que

X        est un groupement acétyle, Boc ou un atome d'hydrogène,

X$^4$        est un groupement benzyle,

X$^5$        est un groupement 2,6-dichlorobenzyle,

X$^5$        est un groupement benzyloxycarbonyle ou 2-chlorobenzyloxycarbonyle,

X$^5$        est un groupement p-toluènesulfonyle ou nitro,

X$^{10}$        est une résine porteuse contenant des groupements benshydryle ou méthylbenzhydryle,

R$^1$        est mis pour D-Nal(2) ou D-Phe(4-Cl),

R$^2$        est mis pour D-Phe(4-Cl),

27

$R^3$      est mis pour D-Trp ou D-Pal(3) et

$R^{10}$    est mis pour D-Ala.

5. Peptide selon l'une ou plusieurs des revendications précédentes, caractérisé en ce que X ou $X_1$ est un groupement acétyle.

6. Peptide selon l'une ou plusieurs des revendications precédentes, caractérisé en ce que X ou $X_1$ est un groupement carbamoyle.

7. Peptide selon l'une ou plusieurs des revendications précédentes, caractérisé en ce que $R^6$ ou $R^{*6}$ est mis pour D-Cit ou D-Hci.

8. Procédé de préparation de peptide de formule

X-$R^1$-$R^2$-$R^3$-Ser-Tyr-$R^6$-Leu-Arg-Pro-$R^{10}$-$NH_2$      I

dans laquelle

X      est un groupement acyle composé de chaînes droites ou ramifiées d'acides carboxyliques aliphatiques ou alicycliques à 1-7 atomes de carbone, ou un groupement carbamoyle,

$R^1$      est mis pour D- ou L-Pro, D- ou L-$\Delta^3$-Pro, D-Phe, D-Phe(4-Hl), D-Ser, D-Thr, D-Ala, D-Nal(1) ou D-Nal(2),

$R^2$      est mis pour D-Phe ou D-Phe(4-Hl),

$R^3$      est mis pour D-Trp, D-Phe, D-Pal(3), D-Nal(1) ou D-Nal(2),

$R^6$      est mis pour D-Cit, D-Hci, D-Cit(Q) ou D-Hci(Q) et

$R^{10}$    est mis pour Gly ou D-Ala,

Hl      désignant un atome de fluor, de chlore ou de brome et

Q      désignant un reste alkyle en $C_1$-$C_3$,

et de sels d'acides d'addition de ces peptides, acceptables pharmaceutiquement,
caractérisé en ce qu'on les prépare par condensation, usuelle dans la chimie des peptides, de fragments de peptide correspondants ou par construction progressive, usuelle dans la chimie des peptides, ou en ce que, dans un peptide de formule I, où $R^6$ est mis pour D-Orn ou D-Lys, on transforme le groupement amino libre de D-Orn ou D-Lys en groupement uréido par réaction avec un cyanate alcalin, un composé générateur de cyanate ou un isocyanate d'alkyle en $C_1$-$C_3$, on acyle éventuellement les peptides obtenus et/ou on les convertit en les amides et on les transforme éventuellement en sels d'acides sans inconvénients physiologiques.

9. Procédé selon la revendication 8, caractérisé en ce qu'on conduit la construction progressive en commençant par fixer par liaison covalente, à un support synthétique usuel à cet effet, l'acide aminé glycine ou D-alanine carboxy-terminal ou ses amides, dont le groupement amino en position α est protégé, on enlève le groupement de protection du groupement α-amino, on fixe, au groupement amino libre ainsi obtenu, l'acide aminé protégé suivant, par son groupement carboxy, puis on enlève à son tour le groupement de protection du groupement α-amino de ce deuxième acide aminé, on y fixe l'acide aminé suivant et on enchaîne de proche en proche de cette manière, dans l'ordre correct, les autres acides aminés du peptide à synthétiser et, après l'enchaînement de tous les acides aminés, on enlève du support le peptide fini selon la revendication 1 et, éventuellement, on enlève d'autres groupements présents de protection de fonctions latérales.

10. Composés selon la revendication 1 ou 2, destinés à être utilisés comme substances thérapeutiques actives.

11. Médicament contenant un composé de formule générale I selon la revendication 1 ou 2, outre des excipients et/ou des diluants ou des adjuvants usuels.

12. Procédé de préparation d'un médicament, caractérisé en ce qu'un composé I selon la revendication 1 ou 2 est transformé en préparation pharmaceutique ou est mis sous une forme utilisable en thérapeutique, avec des excipients et/ou des diluants ou autres adjuvants pharmaceutiques usuels.

**13.** Procédé selon la revendication 11 ou 12, caractérise en ce que la quantité de substance active selon l'une quelconque des revendications 1 à 7 est comprise entre 0.1 et 2,5 mg/kg de poids corporel par jour.

**14.** Utilisation de composés selon la revendication 1 ou 2 pour la préparation de médicaments.